# EUROPEAN PATENT APPLICATION

(11) **EP 2 316 483 A1**
(43) Date of publication of application: **04.05.2011**
(21) Application number: 10196357.7
(22) Date of filing: 01.12.2006
(51) Int. Cl.: A61K 39/39

(54) **Compositions and methods relating to the treatment of cancer and infectious diseases**

(30) Priority: 01.12.2005 IE 20050799
(62) Divisional of application: 06831861.7
(71) Applicant: The Provost Fellows And Scholars Of The College Of The Holy and Undivided Trinity Of Queen Elizabeth Near Dublin, Dublin 2 (IE)
(72) Inventor: Mills, Kingston, Dublin 2 (IE); Toomey, Deirdre, Dublin 2 (IE); Jarnicki, Andrew, Dublin 2 (IE)
(74) Representative: Stark, Gordon Drummond

(57) **Abstract**

The present invention provides methods for modulating an immune response by administering a composition comprising a Toll-like receptor agonist and an immune mediator which downregulates the expression of the anti-inflammatory cytokine IL-10 and upregulates the expression of the pro-inflammatory cytokine IL-12. The methods can be used to provide therapeutic treatment for cancerous conditions and infectious diseases.

## Description

### Field of the Invention

The present invention relates to novel compositions and methods for preventing or treating cancer or malignant conditions and infectious diseases. In particular, the invention relates to a composition and method for promoting the induction of type 1 immune responses (such as Th1 cells) and/or the subversion of regulatory T (Treg) cells. The invention further extends to uses of the compositions of the invention in the treatment of disease.

### Background to the Invention

Cells of the innate immune system, especially dendritic cells (DCs), direct the differentiation of naïve CD4⁺ T cells into functionally distinct Th1, Th2 or regulatory T (Treg) cell subtypes. Activation of immature DCs through binding of conserved microbial molecules to pathogen recognition receptors (PRRs), such as Toll-like receptors (TLR) and integrins, is accompanied by maturation and homing to the lymph nodes, where the mature DCs present antigen (Ag) to the naïve T cells. Activation of DCs by pathogen derived molecules plays a critical role in regulating the differentiation of naïve CD4⁺ T cells into the distinct T cell subtypes. Th1 cells confer protection against intracellular infection and tumours but are also associated with inflammatory responses and autoimmune disease, whereas Th2 cells are involved in allergic responses. Treg cells are capable of suppressing Th1 and Th2 responses.

Tumour progression in normal immunocompetent individuals may reflect a failure of the immune system to recognize tumour antigens or may result from subversion of anti-tumour immune responses through the induction and activation of regulatory T cells, which may be further influenced by the environment of the growing tumour. Innate and adaptive immune responses can be induced against tumours and the protective effector cells include CD8⁺ cytotoxic T lymphocytes (CTL), IFN-gamma producing CD4⁺ and CD8⁺ T cells, NK cells and macrophages. While T cells usually constitute a main immune cell population attracted to the tumour site, they are often ineffective at killing the tumour and evidence suggests that this may result from the function of regulatory T cells (Woo, E. Y., et al.. 2002. Eur J Immunol 32:3267).

Natural CD4⁺CD25⁺ regulatory T cells that express the transcriptional repressor Foxp3 and emerge as mature T cells from the thymus play a critical role in maintaining tolerance to self-antigen and in preventing autoimmunity (Sakaguchi, S. 2000. Cell 101:455-458).

Inducible regulatory T cells secreting lL-10 (termed Tr1 cells) or TGF-beta (termed Th3 cells) are generated in the periphery in response to pathogen and self antigens. Natural and inducible regulatory T cells can be beneficial to the host during infection by preventing pathogen-induced immunopathology. However, it has also been reported that induction or activation of regulatory T cells by pathogens may be a strategy to subvert protective immunity and depletion of CD4⁺CD25⁺ regulatory T cells has been shown to enhance survival during certain infections (Mills, K.H., and P. McGuirk. 2004. Semin Immunol 16:107-117). Depletion of CD4⁺CD25⁺ T cells can also enhance anti-tumour immunity. The balance of effector versus regulatory T cells may also be influenced by the environment of the growing tumour and this may have a bearing on resolution or progression of tumour growth. Thus vaccines or therapies against tumours may be more effective if regulatory T cells can be suppressed allowing the development of stronger effector T cell responses.

### Toll-like receptors

The Toll-like receptor (TLR) superfamily plays a central role in the recognition of invading pathogens and the initiation of an immune response. Thirteen human TLRs have been identified to date. Each recognises a distinct pathogen-associated molecular pattern (PAMP) leading to the activation of a signalling cascade, which in turn activates the transcription factor NF-kappaB and also the mitogen-activated protein kinases (MAPKs), p38, c-jun, N terminal kinase (JNK) and p42/44. TLR-3 and TLR-4 also activate another pathway culminating in the activation of the transcription factor, interferon regulated factor 3 (IRF3), which binds to the interferon sensitive response element (ISRE), inducing a subset of genes including IFN-beta. The TLRs are members of a larger superfamily, called the interleukin-1 receptor (IL-1 R)/TLR superfamily, that also contains the IL-1 R1 subgroup and the TIR domain-containing adaptor subgroup. All three subgroups possess a cytoplasmic Toll/IL-1 receptor (TIR) domain, which is essential for signalling. The TLRs possess extracellular leucine rich repeats, while the IL-1R1 sub-group have extracellular immunoglobin domains. The adaptor molecules are cytoplasmic and contain no extracellular region.

Toll-like receptor (TLR) agonists have been tested in clinical trials as adjuvants for tumour vaccines. The basis of the therapy is that TLR agonists promote type 1 responses, in particular activating IFN-gamma secreting Th1 cells, NK cells and CD8 CTLs. The results to date have demonstrated enhancement of anti-tumour immune responses, but there is no real indication yet that these will prevent tumour growth, although data in mouse models suggest that they may have some efficacy (Miconnet, I., S. et al. 2002. J Immunol 168:1212*).*

### Vaccines

Vaccines against infectious disease generally confer protective (prophylactic) immunity to infection through the generation of antigen-specific effector T cells, which promote neutralizing antibody production and mediate cellular immunity against the pathogen. These responses can be recalled following the generation of memory T and B cells and are influenced quantitatively and qualitatively by adjuvants in the vaccine formulation. Adjuvants may be exogenous immunomodulators added to the antigen formulation or endogenous components of attenuated or inactivated viral or bacterial vaccines, which non-specifically activate innate immune responses through interaction with TLRs and other pathogen recognition receptors. TLR agonists, including LPS, CPG-oligodeoxynucleotides (CpG-ODN), PolylC, as well as whole bacteria, including *Bordetella pertussis* and *Mycobacterium tuberculosis* all have adjuvant activity and promote effector T cell and antibody responses to coinjected antigens.

### Anti-inflammatory Cytokine Inhibitors

Cyclooxygenase-2 (Cox-2) inhibitors are known to be used as therapies with modest efficacy against various cancers. Cox-2 is an enzyme induced by inflammatory stimuli which induces synthesis of prostaglandin E2 (PGE2) in neoplastic tissues. PGE2 is a potent inducer of IL-10. Cox-2 enhances angiogenesis, cell proliferation and makes cells resistant to apoptosis and this may explain the effect of Cox-2 selective inhibitors (such as CELECOXIB and NS-398) on tumour growth.

MAP kinase inhibitors have been tested clinically in inflammatory disease and ERK and p38 have been previously implicated in cell death and survival of tumour cells *in vitro.*

The present inventors have made the surprising discovery that the administration of a specific combination of known modulators of the immune system can result in the efficacy of these modulators being synergistically enhanced, this leading to the identification by the inventors of improved therapeutic compositions which have utility in the treatment and/or prophylaxis of cancerous conditions and infectious diseases by suppressing anti-inflammatory responses which may inhibit pro-inflammatory immune responses.

Regulatory T cells are known to express anti-inflammatory cytokines such as IL-10 and/or TGF-beta, with the expression of such cytokines being known to suppress the immune response. Regulatory T cells also suppress immune responses through cell-to-cell contact. In cases where the suppressed immune response is directed against the development of cancerous cells, the suppression of the immune response may result in an increase in tumour growth. Consequently, an inhibitor of anti-inflammatory cytokines such as IL-10 and/or TGF-beta or of regulatory T cells would be of utility in improving the efficacy of anti-cancer therapies or infectious disease treatments, particularly vaccines.

### Summary of the Invention

According to a first aspect of the present invention there is provided a composition, for the treatment of a condition where an enhancement of a Th1-mediated immune response is desired, said composition comprising:
(i) at least one Toll-like receptor (TLR) agonist; and
(ii) at least one immune modulator which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated.

The suppressed anti-inflammatory cytokine may be IL-10 and/or TGF-beta. The upregulated pro-inflammatory cytokine may be IL-12, or any further pro-inflammatory cytokine such as interferon gamma, IL-1 and TNF-beta.

The upregulation of pro-inflammatory cytokines serves to promote the inflammatory response which is mediated by effector cells such as Th1 cells and CTL (cytotoxic T lymphocytes).

The immune modulator is an inhibitory compound which suppresses or inhibits at least one molecule which is involved in mediating an anti-inflammatory immune response, or which is involved in a pathway which mediates an anti-inflammatory immune response.

An anti-inflammatory response is characterised by the production of the cytokine IL-10 and the production of Treg cells, such as Tr1 or Th3 cells.

Suitably the inhibition in the production of IL-10 and/or TGF-beta and/or the upregulation of IL-12 is modulated in the cells of the innate immune system, for example dendritic cells.

Suitably the immune modulator causes the suppression of IL-10 production or inhibition of IL-10 function. The immune modulator may further cause the suppression or inhibition of function of TGF-beta. This modulation of the cytokine response (IL-10 and/or TGF-beta) and in particular the modulation of the cytokine expression profiles of the cells of the innate immune system serves to inhibit the induction of Treg cells, that is the subset of T cells which has immunosuppressive activity. In addition to the modulation of IL-10, and TGF-beta the compounds of the invention may modulate the expression of further cytokines which are known to induce an anti-inflammatory effect.

The immune modulator compound may further suppress direct cell to cell contact between a regulatory T cell and other cells of the immune system, therein preventing suppression of the pro-inflammatory immune response which is mediated by this mechanism.

By "selective inhibition of function of regulatory T cells" it is meant that the regulatory T cells so inhibited cannot suppress a pro-inflammatory immune response or mediate an anti-inflammatory immune response, particularly by direct cell to cell contact.

As herein defined, the term "upregulation" when used in relation to the increase in the production of a cytokine means that the activity or expression of that cytokine is greater than that observed in resting cells.

As herein defined, the term "suppression" when used in relation to the decrease in the production of a cytokine means that the activity or expression of the cytokine is lower than that observed in resting cells.

As herein defined, the term "inhibition" when used in relation to the inhibtion in the production of a cytokine means that the activity or expression of the cytokine is inhibited or substantially inhibited when compared to the activity or expression level observed in resting cells.

In one embodiment, the at least one Toll-like receptor (TLR) agonist can be any suitable TLR agonist which will be known to the person skilled in the art. The TLR agonist has binding affinity and specificity to the TLR such that its binding serves to activate the TLR and mediate downstream signalling.

The TLR agonist may have binding specificity for at least one of; TLR-2, TLR-3, TLR-4, TLR-5, TLR-7, TLR-8 and TLR-9. Specific examples of suitable TLR agonists include, but are not limited to; Pam3CSK4, Zymosan, PolylC, dsRNA, LPS (lipopolysaccharide), monophosphoryl lipid A (MPL), Flagellin, CpG-ODN (CPG-oligodeoxynucleotides), Imiquimod, R838 and R837. Further, whole bacteria such as *Bordetella pertussis* and *Mycobacterium tuberculosis* may also act as TLR agonists (Toll agonists). Further, suitable analogues to the TLR agonists listed above may also be used, wherein said analogues function to activate at least one Toll-like receptor.

Suitably the immune modulator is a compound which inhibits the function of a downstream mediator of an immune response. Suitably the modulatory effect is selective to the target. The inhibitor would be provided in an amount effective to inhibit the activity of the target. Suitably the activity being inhibited is the ability of the selectively inhibited molecule to participate in signalling and signal modulation which may contribute to an anti-inflammatory response.

In further embodiments the immune modulator may comprise at least one of an inhibitor of at least one of; a MAP kinase protein, Cox-2, ERK, p38 or pI3K.

Suitably the immune response is an anti-inflammatory response or an innate immune response that promotes the induction of regulatory T cells. In one embodiment the immune modulator is an inhibitor of a MAP kinase protein or a MAP kinase pathway, suitably a selective inhibitor. MAP kinases (Mitogen activated kinases) are proteins which are involved with cellular responses, inflammation and growth.

p38 kinase (p38) is a member of the stress activated protein kinase (SAPK) group of MAP kinases. In mammalian cells, the p38 kinase signalling pathway is involved in the response of the cell to stress and is also known to have a role in the upregulation of apoptosis. Activation of p38 kinase is known to increase the production of molecules which are causative of an inflammatory response such as IL-1, TNF and Cox-2.

In one embodiment the inhibitor is a p38 kinase inhibitor. The inhibitor serves to inhibit the function of at least one iso-form of p38 kinase. This inhibitor therefore serves to inhibit the function of the p38 signalling pathway. Preferably the p38 kinase inhibitor is SB203580 or a pharmaceutically acceptable salt or solvate thereof, or an analogue thereof, wherein the analogue has p38 inhibitory activity. Alternatively the inhibitor may be SB220025 or SB239063 or a pharmaceutically acceptable salt or solvate thereof, or an analogue thereof, wherein the analogue has p38 kinase inhibitory activity.

ERK (extracellularly regulated kinase) is a group of MAP kinases which regulate the growth and proliferation of cells.

In one embodiment the inhibitor is an ERK inhibitor, suitably a selective ERK inhibitor. Preferably the ERK inhibitor is U0126 or a pharmaceutically acceptable salt or solvate thereof, or an analogue thereof, wherein the analogue has ERK inhibitory activity. Alternatively the inhibitor may be PD98059 or an analogue thereof, wherein the analogue has ERK inhibitory activity. In a further embodiment the ERK inhibitor is provided in conjunction with a p38 inhibitory compound.

In further embodiments the immune modulator may be an inhibitor of further MAP Kinases such as MEK 1 or MEK 2, as may compounds which inhibit or block transcription factors which may be present in the Th1 signalling pathway at a point downstream from MAP kinases. An example of such a transcription factor would be c-Fos.

In a further embodiment, the immune modulator is an inhibitor of Phosphoinositide kinase-3, suitably a selective inhibitor. Phosphoinositide kinase-3 (pI3K) is a proto-oncogene which regulates cell longevity.

In one embodiment the pI3K inhibitor is LY294002, a pharmaceutically acceptable salt or solvate thereof, or an analogue thereof, wherein the analogue has pI3K inhibitory activity. Alternatively the pI3K inhibitor may be wortmannin (WMN).

In a further embodiment the immune modulator is a Cox-2 (cyclooxygenase 2) inhibitor. Suitable Cox-2 inhibitors include Celecoxib (CELEBREX (NS-398), Pfizer Corporation), BEXTRA (Pfizer Corporation), Rofecoxib (VIOXX, Merck) or pharmaceutically acceptable salts or solvates thereof, or analogues thereof, wherein the analogue has Cox-2 inhibitory activity. Suitably the Cox-2 inhibitor is a selective inhibitor.

The administration of a Cox-2 inhibitor skews the immune response to the Th1 pathway by inhibiting the induction of IL-10 or TGF-beta producing inducible regulatory T cells or Foxp3-expressing natural regulatory T cells. Accordingly the co-administration of a Cox-2 inhibitor along with a TLR agonist in this aspect of the invention may serve to inhibit both natural and induced regulatory T cells.

The inventors have surprisingly observed that the inhibition of Cox-2 suppresses the production of the anti-inflammatory cytokines IL-10 and TGF-beta when administered along with a TLR Agonist. Further a Cox-2 inhibitor was also shown to induce the production of IL-27 mRNA following stimulation with a Toll-like receptor agonist, and further was seen to increase the production of IL-12p40 and IL-12p35 production by dendritic cells while also reducing IL-10 mRNA expression.

The composition may further comprise at least one tumour specific antigen. The tumour specific antigen may be derived from the complex formed between a heat shock protein and an antigenic peptide isolated from a cancerous cell or from an individual with a cancerous condition.

In one embodiment the composition may further comprise a compound that inhibits tumour cell products which function to enhance growth of the cancerous cell. Such a compound may result in prolonged tumour survival, for example due to conferring drug resistance or by conferring resistance to apoptosis. Examples of such compounds would be well known to the person skilled in the art.

As herein defined, a "condition where an enhancement of a Th1-mediated immune response is desired" may be a cancerous or malignant condition, or further, said condition may be an infectious disease. Examples of such conditions are described hereinafter.

A further aspect of the present invention provides a pharmaceutical composition for the enhancement of a Th1 mediated immune response, wherein the composition comprises at least one Toll-like receptor agonist and at least one immune modulator compound which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated along with a pharmaceutically acceptable excipient, diluent or carrier.

In one embodiment the immune modulator selectively inhibits the function of regulatory T cells, such that they cannot suppress a pro-inflammatory immune response which is mediated by cell to cell contact.

In one embodiment the production of the anti-inflammatory cytokine IL-10 is inhibited or suppressed by the modulator while the production of the pro-inflammatory cytokine IL-12 is enhanced.

Suitably the immune modulator is a compound which inhibits the function of a downstream mediator of an immune response, and may in particular be an inhibitor of a MAP kinase protein. The inhibitor may be at least one of; a p38 kinase inhibitor, an ERK inhibitor, an inhibitor of MEK 1 or MEK 2, a pI3K inhibitor or a Cox-2 inhibitor.

Suitable TLR agonists include, but are not limited to; Pam3CSK4, Zymosan, PolyIC, dsRNA, LPS (lipopolysaccharide), monophosphoryl lipid A (MPL), Flagellin, CpG-ODN (CPG-oligodeoxynucleotides), Imiquimod, R838 and R837. Further, whole bacteria such as *Bordetella pertussis* and *Mycobacterium tuberculosis* may also act as TLR agonists (Toll agonists).

In one embodiment the enhancement of the Th1 immune response allows the pharmaceutical composition to be used for the treatment or prophylaxis of a cancerous or malignant condition or of an infectious disease.

In one embodiment the composition may comprise a further modulatory compound which inhibits tumour growth and development or which inhibits or suppresses the function of products or mediators which function to enhance tumour growth. Such a modulator compound may, for example, inhibit or suppress the function of any molecule or pathway which results in prolonged tumour survival. For example, for the modulator compound may inhibit the drug resistance of the tumour cell or inhibit its resistance to apoptosis.

A further aspect of the present invention provides a method for the treatment or prophylaxis of a condition where an enhancement of a Th1-mediated immune response is desired, the method comprising the steps of:
- administering a therapeutically useful amount of at least one Toll-like receptor agonist; and
- administering a therapeutically useful amount of at least one immune modulator which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated.

In one embodiment the production of the anti-inflammatory cytokine IL-10 is inhibited or suppressed by the immune modulator while the production of the pro-inflammatory cytokine IL-12 is enhanced.

Suitably the immune modulator causes the suppression or inhibition of function of at least IL-10. The modulator may further cause the suppression or inhibition of function of TGF-beta. This modulation of the cytokine response and in particular the cytokine expression profiles of the cells of the innate immune system serve to inhibit the induction of Treg cells, that is the subset of T cells which has suppressor activity. In addition to the modulation of IL-10, and TGF-beta the compounds of the invention may modulate the expression of further cytokines or mediators which are known to induce an anti-inflammatory effect.

Further, the immune modulator may mediate an increase in the expression or functional activity of IL-12. The upregulation of further pro-inflammatory cytokines such as IL-1, TNF-beta or IFN-gamma may also be observed.

Suitably the inhibition in the production of IL-10 and/or TGF-beta and/or the upregulation of IL-12 is modulated in the cells of the innate immune system, for example dendritic cells.

Suitably the immune modulator is a compound which inhibits the function of a downstream mediator of an immune response, and may in particular be an inhibitor of a MAP kinase protein. The inhibitor may be at least one of; a p38 inhibitor, an ERK inhibitor, an inhibitor of MEK 1 or MEK 2, a pI3K inhibitor or a Cox-2 inhibitor.

Suitable TLR agonists include, but are not limited to; Pam3CSK4, Zymosan, PolyIC, dsRNA, LPS (lipopolysaccharide), monophosphoryl lipid A (MPL), Flagellin, CpG-ODN (CPG-oligodeoxynucleotides), Imiquimod, R838 and R837. Further, whole bacteria such as *Bordetella pertussis* and *Mycobacterium tuberculosis* may also act as TLR agonists (Toll agonists). In one embodiment, the Toll-like receptor agonist and the immune modulatory compound are co-administered to the subject. Alternatively, the Toll-like receptor agonist and the immune modulator are administered separately or sequentially and in this embodiment the method of administration may be different for the Toll-like receptor agonist and for the immune modulator.

Generally, the subject is a mammal. In a further embodiment, the subject is a human.

The present invention may be used to provide a method for the treatment or prophylaxis of treating any known cancerous or malignant condition. The cancerous or malignant condition may include; fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumour, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumour, cervical cancer, testicular tumour, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, lymphoma, multiple myeloma, Waldenström's macroglobulinemia, and heavy chain disease. Where the compositions, pharmaceutical compositions and methods of the present invention are used for the treatment of infectious diseases, such conditions may include, but are not limited to; Mycobacterium leprae, Mycobacterium tuberculosis, Leishmania, Bordetella pertussis, malaria, influenza virus, HIV, or hepatitis C virus.

A yet further aspect of the invention provides the use of a composition comprising at least one Toll-like receptor agonist and an immune modulator which inhibits the suppression of an immune response through the selective inhibition of function of regulatory T cells or from the modulation of cytokine expression such that at least one anti-inflammatory cytokine is suppressed and at least one pro-inflammatory cytokine is upregulated for the treatment of a condition where an enhancement of a Th1-mediated immune response is desired.

In one embodiment the condition is a malignant or cancerous condition. In a further embodiment the condition is an infectious disease.

In one embodiment the production of the anti-inflammatory cytokine IL-10 is inhibited or suppressed by the immune modulator while the production or the pro-inflammatory cytokine IL-12 is enhanced.

Suitably the immune modulator causes the suppression or inhibition of function of at least IL-10. The modulator may further cause the suppression or inhibition of function of TGF-beta. This modulation of the cytokine response and in particular the cytokine expression profiles of the cells of the innate immune system serve to inhibit the induction of regulatory T cells, that is the subset of T cells which has suppressor activity. In addition to the modulation of IL-10, and TGF-beta the compounds of the invention may modulate the expression of further cytokines or mediators which are known to induce an anti-inflammatory effect. The modulator may also inhibit cell-cell contact suppression by regulatory T cells.

Further, the immune modulator may mediate an increase in the expression or functional activity of IL-12. The upregulation of further pro-inflammatory cytokines such as IL-1, TNF-beta or IFN-gamma may also be observed.

Suitably the inhibition in the production of IL-10 and/or TGF-beta and/or the upregulation of IL-12 is modulated in the cells of the innate immune system, for example dendritic cells.

Suitable TLR agonists include, but are not limited to; Pam3CSK4, Zymosan, PolyIC, dsRNA, LPS (lipopolysaccharide), monophosphoryl lipid A (MPL), Flagellin, CpG-ODN (CPG-oligodeoxynucleotides), Imiquimod, R838 and R837. Further, whole bacteria such as *Bordetella pertussis* and *Mycobacterium tuberculosis* may also act as TLR agonists (Toll agonists).

Suitably the immune modulator is a compound which inhibits the function of a downstream mediator of an immune response, and may in particular be an inhibitor of a MAP kinase protein. The inhibitor may be at least one of; a p38 inhibitor, an ERK inhibitor, an inhibitor of MEK 1 or MEK 2, a pI3K inhibitor or a Cox-2 inhibitor.

A yet further aspect of the invention provides the use of at least one Toll-like receptor agonist and an immune modulator which inhibits the suppression of an immune response through the selective inhibition of function of regulatory T cells or from the modulation of cytokine expression such that at least one anti-inflammatory cytokine is suppressed and at least one pro-inflammatory cytokine is upregulated in the preparation of a medicament for the treatment of a condition where an enhancement of a Th1-mediated or other effector immune response is desired.

In one embodiment the condition is a malignant or cancerous condition. In a further embodiment the condition is an infectious disease.

Suitably the immune modulator is a compound which inhibits the function of a downstream mediator of an immune response, and may in particular be an inhibitor of a MAP kinase protein. The inhibitor may be at least one of; a p38 inhibitor, an ERK inhibitor, an inhibitor of MEK 1 or MEK 2, a pI3K inhibitor or a Cox-2 inhibitor.

Suitable TLR agonists include, but are not limited to; Pam3CSK4, Zymosan, PolyIC, dsRNA, LPS (lipopolysaccharide), monophosphoryl lipid A (MPL), Flagellin, CpG-ODN (CPG-oligodeoxynucleotides), Imiquimod, R838 and R837. Further, whole bacteria such as *Bordetella pertussis* and *Mycobacterium tuberculosis* may also act as TLR agonists (Toll agonists).

In further specific embodiments, the present invention extends to improvements in the efficacy of anti-cancer or melanoma vaccines. A composition which comprises both an anti-cancer vaccine component (such as a tumour specific vaccine) and an immune modulator compound which inhibits regulatory T cells or IL-10 production has been surprisingly identified by the inventors as providing an unexpectedly efficacious composition for the treatment of cancer and malignant conditions.

Vaccination with non-live materials such as proteins generally leads to an antibody response and CD4+ helper T cell response, either Th1 or Th2 responses. On the other hand, vaccination or infection with live materials such as live viruses or intracellular bacteria generally leads to a CD8+ cytotoxic T-lymphocyte (CTL) response. Th1 and CTL responses are crucial for protection against cancers, pathogenic viruses and intracellular bacteria. Th2 responses are crucial for protection against extra cellular bacteria and parasites.

Accordingly, a further aspect of the present invention provides a composition for the treatment of a cancerous or malignant condition comprising;
(i) a composition comprising at least one tumour specific antigen against which an immune response can be mounted, said response being specific for the cancerous or malignant condition,
(ii) at least one Toll-like receptor agonist; and
(iii) an immune modulator which inhibits the suppression of an immune response through the selective inhibition of function of regulatory T cells or from the modulation of cytokine expression such that at least one anti-inflammatory cytokine is suppressed and at least one pro-inflammatory cytokine is upregulated.

Suitably the composition comprising at least one tumour specific antigen is a cancer vaccine, or vaccine component which contains at least one antigen (Ag). Further the tumour specific antigen may be a known tumour antigen which is representative of a specific tumour type.

Suitable TLR agonists and immune modulator compounds are described hereinbefore.

The modulation of the cytokine response and in particular the cytokine expression profiles of the cells of the immune system serve to inhibit the induction of regulatory T cells. This may be mediated by the suppression or inhibition of IL-10.

The cancer vaccine may be any suitable vaccine or vaccine fragment, for example a whole cell vaccine, a DNA vaccine, a sub-unit vaccine or a peptide vaccine.

The immune modulator compound which is co-administered along with the antigen the production of IL-10 may further upregulate the production of the cytokine IL-12. IL-12 serves to skew the immune response down the 'Th1' pathway. In further cases, the inhibitor of IL-10 may not specifically induce IL-12 production; however this may indirectly result from the reduction of production of IL-10.

Typically, the induction of the Th1 immune response is accompanied by the subversion of a regulatory T cell immune response.

A further aspect of the present invention provides a pharmaceutical composition for the treatment or prevention of cancerous or malignant condition, wherein the composition comprises at least one tumour specific antigen against which an immune response can be mounted, at least one TLR agonist and an immune modulatory compound along with a pharmaceutically acceptable excipient, diluent or carrier.

Suitable TLR agonists and immune modulator compounds are described hereinbefore.

In one embodiment the tumour specific antigen is derived from a cancer vaccine, or vaccine component.

A further aspect of the present invention provides a method for the treatment of a cancerous or a malignant condition, the method comprising the steps of:
- administering an anti-cancer vaccine or an antigenic fragment or determinant thereof which comprises at least one tumour specific antigen;
- administering at least one Toll-like receptor agonist, and
- administering a therapeutically useful amount of at least one immune modulator compound which modulates the cytokine response through the inhibition of IL-10 and/or TGF-beta and/or the upregulation of IL-12 by the cells of the innate immune system, to a subject in need thereof.

Suitably the modulator inhibits the suppression of an immune response through the selective inhibition of function of regulatory T cells or from the modulation of cytokine expression such that at least one anti-inflammatory cytokine is suppressed and at least one pro-inflammatory cytokine is upregulated.

Suitable TLR agonists and immune modulator compounds are described hereinbefore.

Generally, the subject is a mammal. In a further embodiment, the subject is a human.

A yet further aspect of the invention provides the use of a composition comprising at least one tumour specific antigen, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated for the treatment of a cancer or malignant condition.

A yet further aspect of the invention provides the use of a composition comprising at least one tumour specific antigen, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated in the preparation of a medicament for the treatment of a cancer or malignant condition.

Suitable TLR agonists and immune modulator compounds are described hereinbefore.

Generally, the subject is a mammal. In a further embodiment, the subject is a human.

In further aspects, the present invention extends to a vaccine composition which comprises a heat shock protein which has been isolated from a cancerous cell or from an individual which has a cancerous condition, and to which an antigenic peptide is non-covalently complexed. This heat shock protein - antigenic peptide complex (HSP-Ag) may be administered to an individual in order to induce an immune response directed to the antigenic peptide. The inventors have surprisingly identified that he co-administration of an immune mediator which serves to inhibit the immune system, such as a Cox-2 inhibitor, results in an enhanced immune response to the antigen which is complexed to the HSP. The isolation of the HSP-Ag complex will allow for the antigen to be processed by antigen presenting cells and presented to the immune system such that an immune response can be mounted in cases where the antigen is recognised as being non-self.

An example of such an HSP-Ag complex would be a complex between the heat shock protein HSP-70 and a tumour-specific peptide. In such an aspect, the present invention would provide a composition for the treatment of a cancerous or malignant condition comprising;
(i) a heat shock protein complexed with an antigenic peptide; and
(ii) an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated.

Suitably the immune modulator modulates the cytokine response through the inhibition of IL-10 and/or TGF-beta and/or the upregulation of IL-12 by the cells of the innate immune system.

The modulation of the cytokine response and in particular the cytokine expression profiles of the cells of the immune system serve to inhibit the induction of regulatory T cells.

Suitable TLR agonists and immune modulator compounds are described hereinbefore.

Suitably the antigen which is complexed to the heat shock protein is a tumour specific antigen and is derived from a cancerous cell.

Where the immune modulator is a Cox-2 inhibitor, the administration of a HSP-70-antigen complex along with a Cox-2 inhibitor results in an enhancement of CD80 expression on dendritic cells.

In one embodiment the HSP-60 / antigen complex is provided in the form of a dendritic cell HSP-70 vaccine.

This aspect of the invention further extends to the use of different heat shock proteins which may be derived from a cancerous cell or a host infected with a cancerous condition. Accordingly, the heat shock protein may be HSP-60, HSP-90 and gp96, but is not limited to the foregoing and may extend to any suitable heat shock protein known to the person skilled in the art.

A yet further aspect of the present invention provides for the use of a heat-shock protein complexed to a tumour specific antigen along with at least one Toll-like receptor agonist and an immune modulator which inhibits the suppression of an immune response through the selective inhibition of function of regulatory T cells or from the modulation of cytokine expression such that at least one anti-inflammatory cytokine is suppressed and at least one pro-inflammatory cytokine is upregulated for use in the treatment of a cancerous or malignant condition.

In a further aspect the invention extends to the use of a heat-shock protein complexed to a tumour specific antigen along with at least one Toll-like receptor agonist and an immune modulator which inhibits the suppression of an immune response through the selective inhibition of function of regulatory T cells or from the modulation of cytokine expression such that at least one anti-inflammatory cytokine is suppressed and at least one pro-inflammatory cytokine is upregulated for the preparation of a medicament for the treatment of a malignant or cancerous condition.

### Dendritic Cell Vaccines

Dendritic cells (DCs) are effective antigen presenting cells (APCs) with the unique ability to prime naive T lymphocytes specific for foreign and self antigens.

Dendritic cells are produced in the bone marrow and migrate via the blood stream into virtually all tissues in the body. These cells are usually found in the structural compartment of such lymphoid organs as the thymus, lymph nodes, and spleen. However, they are also found in the bloodstream and other tissues of the body. Dendritic cells capture antigen when appropriate and migrate to draining lymph nodes where an immune response is initiated.

Dendritic cells process and present peptides via major histocompatibility complex (MHC)-peptide complexes to antigen-specific naive T lymphocytes.

The present invention further extends to the use of the compounds and methods of the invention to induce the production of a specific subset of mature dendritic cells which have a phenotype and cytokine expression profile which promotes an effector T cell response mediated by cell types such as Th1 and CTLs, and which further suppresses the production of regulatory T cells. Both myeloid and lymphoid dendritic cell populations have utility in this invention.

It is desirable to modulate dendritic cells in order to modify their phenotype such that they express a cytokine profile which induces a Th1 cell profile and also a CTL response. The induction of such a subset of dendritic cells serves to avoid the production of semi-mature dendritic cells which can be characterised by their production of IL-10 and TGF-beta, such a cytokine profile resulting in the production of regulatory T cells or high expression of CD80 or CD86 but low expression of CD40.

The present invention further extends to dendritic cell vaccines. The compositions defined hereinbefore can be administered to an individual in order to provide a prophylactic or therapeutic treatment to an individual requiring such therapy in relation to a cancerous condition or an infectious disease. Generally, vaccine compositions which mediate immune responses against infectious diseases are administered prophylactically in order to prevent infection in an individual. There are however exceptions to this, in terms of vaccines to infectious diseases such as hepatitis C and HIV where the vaccine is administered therapeutically. However, in general due to the immuno-compromised state of an individual it is generally undesirable to administer an infectious disease vaccine in a therapeutic manner.

When a vaccine is administered in relation to an individual with a cancerous condition, the vaccine is generally administered therapeutically as the vaccine will comprise a tumour cell or an antigen derived therefrom.

The inventors have identified the utility of dendritic cell vaccines in relation to the treatment of cancerous conditions. Further, the inventors have surprisingly identified that the administration of a vaccine composition comprising a dendritic cell, a Toll-like receptor agonist and an immune modulator provides a therapeutic with enhanced efficacy.

Accordingly a further aspect of the present invention extends to a vaccine composition for the treatment of a cancerous condition comprising:
- a dendritic cell,
- at least one tumour cell antigen,
- at least one Toll-like receptor agonist, and
- an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated.

Suitable TLR agonists and immune modulator compounds are described hereinbefore.

Suitably the dendritic cells will be provided by adoptive transfer of dendritic cells which have been pulsed with the tumour specific antigen in the presence or absence of a Toll-like receptor agonist and an immune modulator compound which serves to inhibit IL-10 can serve to induce Th1 cell response which can be effective against a cancer or malignant condition or an infectious disease. Suitably the dendritic cell is of a semi-mature phenotype.

In one embodiment the at least one tumour antigen may be provided by the administration of a heat shock protein complexed with an antigen. The heat shock protein complexed with the antigenic protein may be derived from a cancerous cell or from an individual having a cancerous condition. A further aspect of the present invention provides a method for the treatment of a cancerous or a malignant condition, the method comprising the steps of:
- administering a dendritic cell which has been pulsed in the presence of a tumour specific antigen,
- administering at least one Toll-like receptor agonist, and
- administering a therapeutically useful amount of at least one immune modulator which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated,
   to a subject in need thereof.

Suitable TLR agonists and immune modulator compounds are described hereinbefore.

A yet further aspect of the present invention provides for the use of a dendritic cell, at least one tumour specific antigen, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated for the treatment of a cancerous condition.

A yet further aspect of the present invention provides for the use of a dendritic cell, at least one tumour specific antigen, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated in the preparation of a vaccine composition for the treatment of a cancerous condition.

The inventors have further identified that a dendritic cell vaccine can be administered to an individual for the treatment of a cancerous or malignant condition wherein a tumour specific antigen is not provided in the composition, but wherein the antigen is a tumour specific antigen derived from the individual to whom the dendritic vaccine composition is administered.

Accordingly, a further aspect of the present invention provides a vaccine composition for the treatment of a cancerous condition comprising:
- a dendritic cell,
- at least one Toll-like receptor agonist, and
- an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated.

Suitable TLR agonists and immune modulator compounds are described hereinbefore.

A further aspect of the present invention provides a method for the treatment of a cancerous or a malignant condition, the method comprising the steps of:
- administering a dendritic cell which has been pulsed in the presence of a tumour specific antigen, and
- administering a therapeutically useful amount of at least one immune modulator which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated,
   to a subject in need thereof.

Suitable immune modulator compounds are described hereinbefore.

A yet further aspect of the present invention provides for the use of a dendritic cell, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated for the treatment of a cancerous condition.

A yet further aspect of the present invention provides for the use of a dendritic cell, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated in the preparation of a vaccine composition for the treatment of a cancerous condition.

Accordingly a yet further aspect of the present invention provides a method for inducing a Th1 response in a subject suitable for the treatment of a cancer or an infectious disease, the method comprising the steps of:
- exposing isolated dendritic cells to a disease specific antigen in the presence of vaccine and or a TLR agonist and an immune modulator compound which inhibits the production of IL-10 and/or TGF-beta and/or upregulates IL-12 production by the cells of the innate immune system *ex-vivo* in order to cause maturation of the dendritic cells to a phenotype that promotes effector cell function,
- administering the dendritic cells to a subject whereby the immune response generated in the subject is sufficient to prevent the onset or progression of cancer or to prevention infection with a pathogenic micro-organism and thereby prevent an infectious disease.

In one embodiment of this aspect of the invention the dendritic cells are autologous to the subject. In one embodiment the dendritic cells are mature dendritic cells.

Suitably the disease specific antigen is a tumour specific antigen.

Suitable TLR agonists and immune modulator compounds are described hereinbefore.

A yet further aspect relates to the use of dendritic cells which have been exposed *ex vivo* to vaccine and/or a TLR agonist and an immune modulator compound which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated in the preparation of a medicament for the treatment of cancer or an infectious disease.

In an alternative embodiment of this aspect of the invention the dendritic cells may be replaced by any other suitable antigen presenting cell. Such suitable antigen presenting cells are macrophages, monocytes and B-cells. In a further embodiment, the modulation of the dendritic cells occurs *ex-vivo.*

In a preferred example the antigen presenting cells, preferably dendritic cells which are re-administered are autologous to the subject. Typically the subject is a human.

In a further aspect of the present invention there is provided a composition for the prophylaxis or treatment of a cancer, malignant condition or infectious disease, said composition comprising;
(i) a dendritic cell which is pulsed in the presence of a vaccine, or vaccine component against which an immune response can be generated specific for an infectious disease, a cancer or a malignant condition,
(ii) an adjuvant such as a toll-like receptor (TLR) agonist; and
(iii) a compound which which inhibits the suppression of an immune response, wherein this suppression results from the selective inhibition of function of regulatory T cells or which causes a modulation of cytokine expression such that at least one anti-inflammatory cytokine is suppressed and at least one pro-inflammatory cytokine is upregulated; and pharmaceutically acceptable excipient, diluent or carrier.

Suitably the inhibition in the production of IL-10 and/or TGF-beta and/or the upregulation of IL-12 is modulated in the cells of the innate immune system, for example dendritic cells.

In one embodiment, the dendritic cell is treated with a whole cell cancer vaccine, such as heat shocked irradiated B16 tumour cells, along with the TLR agonist CpG along with a compound which acts as an inhibitor against the MAP kinase, ERK. In further embodiments, the ERK inhibitor may be replaced with a p38 inhibitor, a pI3K inhibitor or a Cox-2 inhibitor.

In a further embodiment, DCs may be pulsed with HSP-70 derived from caner cells or cell infected with an infectious disease, said DCs further being exposed to a compound which inhibits the production of anti-inflammatory cytokine, one specific example being the administration of a Cox-2 inhibitor, which may serve to inhibit IL-10 and/or inhibit expression of natural Tregs.

### Detailed description of the Invention

Naïve CD4⁺ T cells can differentiate into effector Th1 or Th2 cells or Treg cells and the selective induction of these distinct subtypes appears to be determined by a number of factors, including the maturation status of dendritic cells (DC) and regulatory cytokines secreted by cells of the innate immune system. Certain pathogen-derived immunomodulatory molecules, such as TLR ligands promote the induction of Th1 cells. Other, pathogen-derived molecules, such as FHA and CyaA from *B. pertussis* and CT, promote the induction of Tr cells, either selectively or with Th2 cells. The induction of Treg cells simultaneously with effector T cells may be a host protective strategy to control excessive Th1 or Th2 responses and thereby limit infection-induced inflammation and immunopathology. The induction of Th1 cells by TLR ligands has been linked to their ability to promote IL-12, and IL-27 production from innate cells, especially macrophages and DC. However, TLR ligands have also been shown to induce IL-10 production from macrophages and the Th2-promoting TLR-2 agonist Pam3Cys was shown to induce IL-10 production from DC.

The inventors of the present invention have recently demonstrated that CD4⁺ and CD8⁺ Treg cells that express IL-10 and TGF-beta may subvert anti-tumour immunity and promote tumour growth. They demonstrated that T cell responses to a bystander antigen were suppressed in mice immunized subcutaneously with the antigen in the region of growing CT26 tumour. T cells in the growing tumour expressed mRNA for IL-10, TGF-beta and Foxp3 and a high frequency of CD4⁺ and CD8⁺ T cells infiltrating the tumour or in draining lymph nodes secreted IL-10, but a lower frequency secreted IFN-gamma. IL-10-secreting macrophages and dendritic cells also infiltrated the growing tumour. CD8⁺ CTL responses were undetectable in mice with lung metastases and weak and transient following subcutaneous injection of CT26 colon carcinoma cells, but were enhanced in the presence of anti-IL-10 and anti-TGF-beta. Depletion of CD4⁺ or CD25⁺ T cells in vivo significantly enhanced IFN-gamma production by CD8⁺ T cells, reduced subcutaneous tumour volume and lung metastases and enhanced survival. In contrast, removal of CD8⁺ T cells abrogated CTL responses and promoted progression of the subcutaneous tumour, but reduced lung metastases. These findings suggest that tumour growth facilitates the induction or recruitment of CD4⁺ Treg cells that secrete IL-10 and TGF-beta and suppress effector CD8⁺ T cell responses. However CD8⁺ Treg cells expressing IL-10 and TGF-beta are also recruited or activated by the immunosuppressive environment of the lung, where they may suppress the induction of anti-tumour immunity. The inventors of the present invention have previously demonstrated that administration of TLR agonists simultaneously induces IL-10 secreting Treg cells and Th1 cells. TLR ligands induced T cells that secrete IFN-gamma, IFN-gamma and IL-10 or IL-10 only. Furthermore, these distinct populations of IL-10-secreting Tr1 and IL-10 and IFN-gamma secreting Th1-like Treg cells suppressed IFN-gamma production by Th1 cells, suggesting that TLR ligands simultaneously induce distinct populations of regulatory and effector T cells. It was previously reported that innate IL-12 and IL-10 direct the induction of Th1 and Treg1 cells respectively. The inventors demonstrated that TLR-2, TLR-4, TLR-5, TLR-7, TLR-8 and TLR-9 ligands activated IL-10 as well as IL-12 production from DC. Furthermore, TLR ligands induced phosphorylation of ERK and p38 MAP kinases and inhibitors of ERK and P38 suppressed TLR ligand-induced IL-10 and enhanced IL-12 production from dendritic cells.

Without wishing to be bound by theory, the inventors of the present invention believe that p38 inhibitors, pI3K inhibitors, ERK inhibitors and/or Cox-2 inhibitors enhance the efficacy of the TLR agonist and/or the HSP70 vaccine by suppressing innate cell (e.g. dendritic cell) IL-10 production and enhancing IL-12, thereby promoting the induction of Th1 and CTL, but not Treg cells. This limits the subversion of anti-tumour immune responses associated with the induction and activation of Treg cells.

### Definitions

As used herein, the term "T cell" includes CD4+ T cells and CD8+ T cells. The term T cell also includes both T helper 1 type T cells and T helper 2 type T cells and cytotoxic T lymphocytes (CTL).

A "subject" in the context of the present invention includes and encompasses mammals such as humans, primates and livestock animals (e.g. sheep, pigs, cattle, horses, donkeys); laboratory test animals such as mice, rabbits, rats and guinea pigs; and companion animals such as dogs and cats.

### Treatment / Therapy

The term 'treatment' is used herein to refer to any regimen that can benefit a human or non-human animal. The treatment may be in respect of an existing condition or may be prophylactic (preventative treatment). Treatment may include curative, alleviation or prophylactic effects.

More specifically, reference herein to "therapeutic" and "prophylactic" treatment is to be considered in its broadest context. The term "therapeutic" does not necessarily imply that a subject is treated until total recovery. Similarly, "prophylactic" does not necessarily mean that the subject will not eventually contract a disease condition.

Accordingly, therapeutic and prophylactic treatment includes amelioration of the symptoms of a particular condition or preventing or otherwise reducing the risk of developing a particular condition. The term "prophylactic" may be considered as reducing the severity or the onset of a particular condition. "Therapeutic" may also reduce the severity of an existing condition.

### Administration

The active ingredients used in the present invention can be administered separately to the same subject or can be co-administered as a pharmaceutical composition. The pharmaceutical composition will generally comprise a suitable pharmaceutical excipient, diluent or carrier selected depending on the intended route of administration.

The active ingredients (optionally in the form of a pharmaceutical composition) can be administered to a patient in need of treatment via any suitable route. The precise dose will depend upon a number of factors, as is discussed below in more detail.

One suitable route of administration is parenterally (including subcutaneous, intramuscular, intravenous, by means of, for example a drip patch). Other suitable routes of administration include (but are not limited to) oral, rectal, nasal, topical (including buccal and sublingual), infusion, vaginal, intradermal, intraperitoneal, intracranial, intrathecal and epidural administration or administration via oral or nasal inhalation, by means of, for example a nebuliser or inhaler, or by an implant.

For intravenous injection, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection, Lactated Ringer's injection. Preservatives, stabilisers, buffers, antioxidants and/or other additives may be included, as required.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

The composition may also be administered via microspheres, liposomes, other microparticulate delivery systems or sustained release formulations placed in certain tissues including blood. Suitable examples of sustained release carriers include semipermeable polymer matrices in the form of shared articles, e.g. suppositories or microcapsules. Implantable or microcapsular sustained release matrices include polylactides (US Patent No. 3,773,919 or European Patent Application No 0,058,481) copolymers of L-glutamic acid and gamma ethyl-L-glutamate (Sidman et al, Biopolymers 22(1): 547-556, 1985), poly (2-hydroxyethyl-methacrylate) or ethylene vinyl acetate (Langer et al, J. Biomed. Mater. Res. 15: 167-277, 1981, and Langer, Chem. Tech. 12:98-105, 1982).

Examples of the techniques and protocols mentioned above and other techniques and protocols which may be used in accordance with the invention can be found in Remington's Pharmaceutical Sciences, 18th edition, Gennaro, A.R., Lippincott Williams & Wilkins; 20th edition (December 15, 2000) ISBN 0-912734-04-3 and Pharmaceutical Dosage Forms and Drug Delivery Systems; Ansel, H.C. et al. 7th Edition ISBN 0-683305-72-7 the entire disclosures of which are herein incorporated by reference.

### Pharmaceutical Compositions

As described above, the present invention extends to a pharmaceutical composition for the treatment of cancerous or malignant condition and/or an infectious disease and, in particular, for the induction of a Th1 immune response and the suppression or inhibition of a Treg immune response. Pharmaceutical compositions according to the present invention, and for use in accordance with the present invention may comprise, in addition to active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. The formulation may be a liquid, for example, a physiologic salt solution containing non-phosphate buffer at pH 6.8-7.6, or a lyophilised or freeze dried powder.

### Dose

The composition is preferably administered to an individual in a "therapeutically effective amount" or a "desired amount", this being sufficient to show benefit to the individual.

As defined herein, the term an "effective amount" means an amount necessary to at least partly obtain the desired response, or to delay the onset or inhibit progression or halt altogether the onset or progression of a particular condition being treated.

The amount varies depending upon the health and physical condition of the subject being treated, the taxonomic group of the subject being treated, the degree of protection desired, the formulation of the composition, the assessment of the medical situation and other relevant factors. It is expected that the amount will fall in a relatively broad range, which may be determined through routine trials.

Prescription of treatment, e.g. decisions on dosage etc, is ultimately within the responsibility and at the discretion of general practitioners, physicians or other medical doctors, and typically takes account of the disorder to be treated, the condition of the individual patient, the site of delivery, the method of administration and other factors known to practitioners.

The optimal dose can be determined by physicians based on a number of parameters including, for example, age, sex, weight, severity of the condition being treated, the active ingredient being administered and the route of administration.

A broad range of doses may be applicable. Considering a patient, for example, from about 0.1 mg to about 1 mg of agent may be administered per kilogram of body weight per day. Dosage regimes may be adjusted to provide the optimum therapeutic response. For example, several divided doses may be administered daily, weekly, monthly or other suitable time intervals or the dose may be proportionally reduced as indicated by the exigencies of the situation.

Unless otherwise defined, all technical and scientific terms used herein have the meaning commonly understood by a person who is skilled in the art in the field of the present invention.

Throughout the specification, unless the context demands otherwise, the terms 'comprise' or 'include', or variations such as 'comprises' or 'comprising', 'includes' or 'including' will be understood to imply the inclusion of a stated integer or group of integers, but not the exclusion of any other integer or group of integers.

The present invention will now be described with reference to the following examples which are provided for the purpose of illustration and are not intended to be construed as being limiting on the present invention, and further, with reference to the figures.

### Brief description of the figures

Figure 1 shows CT26 tumour cells inhibit T cell proliferation and IFN-gamma production to a bystander antigen;
Figure 2 shows CT26 tumour cells secrete TGF-beta in vitro and IL-10 in vivo;
Figure 3 shows enhanced IL-10, TGF-beta and Foxp3 mRNA expression in tumour infiltrating T cells;
Figure 4 shows IL-10 production by CD4⁺ and CD8⁺ T cells, macrophages and dendritic cells at the tumour site;
Figure 5 shows tumour supernatants induce Cox-2 expression in dendritic cells and macrophages;
Figure 6 shows CpG induces Cox-2 expression in DC and peritoneal macrophages;
Figure 7 shows Th1-promoting TLR ligands also induce IL-10 secreting T cells;
Figure 8 shows TLR ligands promote the induction of regulatory T cells;
Figure 9 shows TLR ligands stimulate IL-10 and IL-12 production from DC;
Figure 10 shows TLR ligands-induced IL-10 production in DC is mediated through activation of ERK and p38 MAP kinases;
Figure 11 shows that a p38 inhibitor, alone or in combination with an ERK inhibitor, suppresses CpG-induced IL-10, while increasing IL-12 production;
Figure 12 shows addition of p38 inhibitor, alone or with an ERK inhibitor, to dendritic cells stimulated with foreign antigen and CpG enhances their ability to induce Th1 and suppress the induction of IL-10-secreting T cells;
Figure 13 shows addition of p38, ERK and Cox-2 inhibitors to dendritic cells stimulated with foreign antigen and CpG enhances their ability to induce Th1 and suppress the induction of IL-10-secreting T cells;
Figure 14 shows inhibition of Cox-2 suppresses CpG induced IL-10 and TGF-beta in vivo;
Figure 15 shows inhibition of Cox-2 enhances CpG-induced IL-27 mRNA while reducing IL-10 mRNA in the draining lymph node;
Figure 16 shows inhibition of Cox-2 suppresses CpG-induced IL-10 while increasing IL-12p40 production by DC;
Figure 17 shows inhibition of Cox-2 enhances CpG-induced IL-27 mRNA while reducing IL-10 mRNA expression in DC;
Figure 18 shows inhibition of Cox-2 enhances CpG induced IL-12p35 and IL-12p40 mRNA expression, while reducing IL-10 mRNA expression in DC;
Figure 19 shows therapy with a combination of Cox-2 inhibitor and CpG reduces tumour growth;
Figure 20 shows a Cox-2 inhibitor enhances the therapeutic effect of CpG on tumour growth;
Figure 21 shows therapy with a combination of Cox-2 inhibitor and CpG enhances survival of mice after tumour challenge;
Figure 22 shows Cox-2 or ERK inhibitors enhance the therapeutic efficacy of dendritic cell tumour vaccine;
Figure 23 shows Cox-2 inhibitor enhances the therapeutic efficacy of a dendritic cell HSP-70 vaccine;
Figure 24 shows the combination of Cox-2 inhibitor and HSP70 vaccine enhances CD80 expression on DC;
Figure 25 shows inhibition of ERK suppresses TLR agonist induced PGE₂ in dendritic cells;
Figure 26 shows a Cox-2 inhibitor suppresses TLR agonist induced PGE₂ and IL-10 production by dendritic cells;
Figure 27 shows inhibition of p38 reduces CpG-induced PGE₂ production;
Figure 28 shows inhibition of p38 suppresses IL-10 and enhances IL-12 production by tumour vaccine and TLR agonist-stimulated dendritic cells;
Figure 29 shows inhibition of p38 enhances therapeutic efficacy of a dendritic cell vaccine;
Figure 30 shows inhibition of p38 enhances the therapeutic efficacy of dendritic cells pulsed with a tumour vaccine and CpG;
Figure 31 shows enhanced survival of mice following therapeutic administration of dendritic cell vaccine pulsed with a tumour cell vaccine in the presence of a p38 inhibitor;
Figure 32 shows therapy with dendritic cells pulsed with a whole tumour vaccine and CpG enhances T cell IFN-gamma production in the tumour;
Figure 33 shows therapy with dendritic cells treated with B16 vaccine, CpG and a p38 inhibitor enhances the frequency of CD4⁺ T cells but decreases the frequency of regulatory T cells within the tumour mass;
Figure 34 shows pertumoural injection of CpG and a p38 inhibitor increases survival;
Figure 35 shows a pI3K inhibitor enhances therapeutic efficacy of dendritic cells pulsed with a tumour vaccine and CpG;
Figure 36 shows transfer of dendritic cells pulsed with a tumour vaccine, CpG and a pI3 kinase inhibitor enhances survival of mice with growing tumours;
Figure 37 shows a p38 inhibitor with CpG as adjuvant enhances the protective efficacy of an acellular pertussis vaccine;
Figure 38 shows a p38 inhibitor enhances IFN-gamma and reduced IL-10 in responses to an acellular pertussis vaccine formulated with CpG as the adjuvant;
Figure 39 shows a p38 inhibitor reduces IL-10 in response to an acellular pertussis vaccine formulated with CpG as the adjuvant;
Figure 40 shows addition of a p38 inhibitor to an acellular pertussis vaccine formulated with CpG as the adjuvant enhances local IL-1 beta after *B. pertussis* challenge of mice;
Figure 41 shows that therapeutic immunization with a tumor vaccine, TLR agonist and a p38 inhibitor reduces B16 tumor growth in mice, and
Figure 42 shows that tumor growth is not significantly exacerbated in IL-10-defective mice.

### EXAMPLES

### Mice

BALB/c and C57BL/6 mice were purchased from Harlan UK Ltd (Bicester, Oxon, U.K.). DO.11.10 OVA T cell Receptor (TCR) transgenic (Tg) mice were bred in house. All mice were maintained under specific pathogen free conditions and according to the regulations and guidelines of the Irish Department of Health.

### Tumour lines

The CT26 colon carcinoma-derived cell line was maintained in RPMI 1640 supplemented with 10% heat inactivated FCS and from solid tumours in BALB/c mice when challenged sub-cutaneously (s.c.). The B16F10 tumour cell line was maintained in DMEM supplemented with 10% heat inactivated FCS and forms solid tumours in C57BL/6 mice when challenged s.c. Mice were injected with 2 x 10⁵ tumour cells in both experimental models unless otherwise stated.

### Peritoneal macrophages

Peritoneal cavities were washed with 5 ml of warm media. Cells were plated in petri dishes, and the non-adherent cells removed after 2 hours. Adherent cells were then plated at 10⁶ per ml.

### Heat shocked- irradiated B16 tumour cells

B16 tumour cells were incubated at 43°C for 1 hour. Cells were then irradiated at 20K Gy, and then incubated at 37°C for 4 hours. Cells were added to BMDC at 1:1 ratio.

### Influence of tumour on proliferation and cytokine responses to an unrelated antigen

D011.10 mice were injected s.c. in the flank with 200 µg OVA alone or with 2x10⁵ CT26 cells. Mice were boosted 7 days later with 200 µg OVA and 14 days later lymph node cell suspensions were prepared and cultured at 2 x 10⁶/ml in 96 microtitre round bottom plates with OVA (50, 150 and 500 µg/ml) at 37°C in 5% CO₂. After 3 days supernatants were removed and IFN-gamma concentrations were determined by two-site ELISA. After 4 days of culture, 2 µCi ³H-thymidine (Amersham) was added to each well. Plates were incubated for a further 6 hours, after which cells were harvested (Tomtec Harvester 96 Mach III M) onto filtermats and cpm were determined using a beta-counter (1450 Microbeta Trilux, Wallac).

### Immunisation

Mice were immunised s.c. with keyhole limpet haemocyanin (KLH; 5 µg), KLH and CpG-ODN (25 µg), LPS (20 µg), PolyIC (25 µg), killed *B*. *pertussis* (1 X 10⁹), or cholera toxin (CT) (10 ng).

### Ex vivo cytokine responses

C57BL/6 mice were injected into the flank with either CpG (10 µg) or with the Cox-2 inhibitor Celecoxib (100 µg) in 100 µl. The draining inguinal lymph nodes were removed after 2 and 6 hours, passed through a sieve and resuspended in 1 ml of PBS. S/N was measured for IL-10 and TGF-beta by ELISA, while mRNA was extracted from the cells and IL-10 and IL-27p28 measured by RT-PCR.

### Antigen-specific cytokine production

Lymph node or spleen cells (2 x 10⁶/ml) removed 7 days after immunization were cultured with KLH (50 µg/ml), sonicated *B. pertussis (5* µg/ml) or medium only. Supernatants were removed after 72 hours and IL-4, IL-10 and IFN-gamma concentrations determined by ELISA.

### Antigen-specific T cell lines

KLH-specific CD4⁺T cell lines were established from spleen or lymph nodes of mice immunized with KLH and CpG-ODN by stimulating spleen or lymph node cells with antigen (10 µg/ml KLH) and antigen presenting cells. In certain cases anti-IFN-gamma or IL-12 and anti-IL-10 were added at the initiation of the cultures. T cell lines established after 2-3 rounds of antigen re-stimulation were cultured with KLH (10 µg/ml) and APC; IFN-gamma, IL-4 and IL-10 concentrations in supernatants determined by ELISA 3 days later. Lymph node cells were cultured with antigen (KLH or in some experiments *B. pertussis* antigen) and after 6 days cells for intracellular staining.

### Suppressor assay

Spleen cells from mice immunized with KLH and CpG-ODN were cultured with KLH in the presence of IL-12 to generate a KLH-specific Th1 type T cell line. A Tr1-type cell line that secreted IL-10 and low or no IFN-gamma or IL-4 was established from mice immunized with KLH and CpG by initial culture in the presence of anti-IFN-gamma. A Th1 Tr-type T cell line that expressed IFN-gamma and IL-10 was established by culturing spleen cells from mice immunized with KLH and CpG with antigen and APC without the addition of cytokines or antibodies. The Th1 cell line (1X10⁵/ml) was cultured with APC (irradiated spleen cells; 2X10⁶/ml) and KLH alone or in the presence of Tr1 or Th1 Tr cells at ratio of 1:3. 1:1 or 3:1. Supernatants were removed after 3 days and the concentration of IFN-gamma was tested by ELISA. Results are expressed relative to the response of the Th1 cell alone.

### DC activation

Bone-marrow derived immature DC (BMDC) were generated by cultivation of extracted bone marrow cells with GM-CSF (40ng/ml) for 10 days. BMDC from either BALB/c or C57BL/6 mice were incubated with 1 ng/ml - 10 µg/ml of the TLR agonists, Pam3CSK4, Zymosan, LPS, Flagellin, PolyIC, CpG-ODN or medium only. In inhibition experiments, activated cells were pretreated (1 hour) with or without the MEK 1/2 (ERK) inhibitor U0126 (1.25 - 5 µM), Cox-2 inhibitor NS-398 (0.1 - 10 µM) or p38 inhibitor SB203580 (0.1 - 10 µM). BMDC were also treated with various concentrations of B16 supernatants (S/N) for 24 hours. B16 S/N was prepared by growing 5 x 10⁵ B16 cells/ml for 1 week and then removing the spent media. After 6 or 24 hours supernatants were removed and cytokine concentrations were determined by ELISA, or cells were homogenised, and resultant mRNA was analysed by RT-PCR.

### DC transfer experiments

Activated BMDC from C57BL/6 mice were incubated with CpG (5 µg) and KLH (5 µg) and pretreated with combinations of inhibitors to Cox-2 (NS-398:5 µM), p38 (SB203580:1 µM) and ERK (U0126:5 µM). 2.5 x 10⁵ treated cells were injected into each footpad. Popliteal lymph nodes and spleens were removed after 5 days, and single cell suspensions were restimulated with KLH (2, 10, 50 µg/ml). S/N were removed after 3 days, and aliquots were measured for IFN-gamma and IL-10 by ELISA.

For tumour experiments, C57BL/6 mice were challenged with 2 x 10⁵ B16 tumour cells s.c. and treated with 3 injections s.c. of treated BMDC (1-5 x 10⁵) one week apart, starting on day 3 into the tumour site. BMDC were loaded with heat shocked:irradiated B16 tumour cells with CpG (5 µg/ml) or with either the Cox-2 inhibitor Celecoxib (5 µM) or the ERK inhibitor U0126 (5 µM) for 24 hours. Mice were routinely monitored for tumour growth.

### In vitro activation of T cells with modulated DC

DC from BALB/c were incubated with OVA Class II peptide (5 µg). Concurrently, cells were stimulated with either CpG (10 µg) or with the p38 inhibitor SB203580 (1 or 5 µM) and with the ERK inhibitor U0126 (5 µM) for 24 hours. D011.10 OVA-TCR T cells were then added to the BMDC (10:1). Supernatants were removed after 3 and 11 days, and aliquots were measured for IL-10 and IFN-gamma by ELISA.

### Direct tumour therapy

C57BL/6 mice were challenged with 2 x 10⁵ tumour cells s.c. and injected s.c. on days 3, 5 and 7 with either CpG (10 or 20 µg), the Cox-2 inhibitor Celecoxib (100 or 500 µg) or a combination of both at the tumour site. Mice were routinely monitored for tumour growth and survival. Tumour size was measured in two dimensions by callipers and determined by the following formula: (width² x length) x π/6 where width is the lesser value. Mice were killed when tumour a length measured greater than 15 mm.

### Western Blot analysis

DC were cultured at 1x10⁶/ml with TLR ligands for 15 minutes to 8 hours. In certain experiments, the ERK or p38 inhibitors were added 1 hr before the TLR ligands. Cell lysates were resolved by SDS-PAGE, transferred to nitrocellulose membranes and blotted with antibodies specific for phospho-p38 (pp38; Cell Signal Technology, MA, USA), or phospho-ERK (pERK; Santa Cruz Biotechnologies, USA) and a horseradish peroxidase-linked secondary antibody. The nitrocellulose was stripped and probed with antibodies specific for total p38 or total ERK.

### T cell purification by magnetic cell sorting

Cell suspensions were prepared from lymph nodes, lungs or surgically removed tumours. Tissue samples were washed with medium, finely chopped with a scalpel blade and incubated with 0.1 % solution of collagenase D (Sigma) in Hanks balanced salt solution for 30 mins at 37°C. Cells suspensions were then passed through a 70 µm cell strainer and red blood cells were lysed. Cells were washed, counted and resuspended in 90 µl of MACS buffer with 10 µl of anti-CD4 or anti-CD8 magnetic beads (Miltenyi Biotech) and incubated at 4-8°C for 15 minutes. MACS buffer (2 ml) was added to each sample and then centrifuged at 300 x g for 10 minutes. Each sample was then resuspended in 500 µl of MACS buffer and sorted using an AutoMacs (Miltenyi Biotech).

### Flow cytometric analysis and intracellular cytokine staining

Single cell suspensions were prepared from lymph nodes, lungs and tumours. Lungs and tumours were digested in Hanks balanced salt solution with 0.1 % collagenase D (Sigma). Cells were stimulated with PMA (10 ng/ml) and ionomycin (1 µg/ml) for 1 hour, then Brefeldin A (10 µg/ml) was added for 4 hours at 37°C. Cells were resuspended with antibodies specific for either CD4 (Caltag), CD8 (BD Pharmingen), CD11 c (BD Pharmigen) or F4/80 (Caltag). Cells were then fixed with 50 µl of fixation medium A (Fix & Perm cell permeabilization kit, Caltag Laboratories). Cells were incubated with 50 µl of the permeabilization medium B (Fix & Perm cell permeabilization kit, Caltag Laboratories) and 5 µl of anti-IL-10 or anti-IFN-gamma antibodies (BD Pharmingen) and immunofluorescence analysed using CELLQuest™ software on a FACScalibur™ (Becton-Dickson, San Jose, CA).

### Reverse Transcriptase-PCR

RNA was extracted from either spleen, lung, LN or tumour cells or purified CD4⁺ and CD8⁺ T cells using TriReagent (Sigma Aldrich) and reverse transcribed using Superscript II RT (Invitrogen) and OligodT₍₁₂-₁₈₎ primers (Invitrogen).
Primers specific for murine TGF-beta (sense-AGACGGAATACAGGGCTTTCGATTCA, anti-sense-CTTGGGCTTGCGACCCACGTAGTA) IL-10 (sense-CTGGACAACATACTGCTAACCGAC, anti-sense-TTCATTCATGGCCTTGTAGACACC), Foxp3 (sense-CAGCTGCCTACAGTGCCCCTA, anti-sense CATTTGCCAGCAGTGGGTAG) Cox-2 (sense-GTATCAGAACCGCATTGCCTCTGA, anti-sense CGGCTTCCAGTATTGAGGAGAACAGAT), IP-10 (sense-CGCACCTCCACATAGCTTACAG, anti-sense-CCTATCCTGCCCACGTGTTGAG, IL-23p19 (sense-TCTCGGAATCTCTGCATGC, anti-sense-CTGGAGGAGTTGGCTGAGTC), IL-15 (sense-CATATGGAATCCAACTGGATAGATGTAAGATA, antisense-CATATGCTCGAGGGACGTGTTGATGAACAT) and beta-actin (sense-GGACTCCTATGTGGGTGACGAGG, anti-sense-TGCCAATAGTGATGACTTGGC) were used with 2 µg of sample cDNA and amplified with Taq polymerase (Promega) using a Peltier Thermal Cycler.

Heat shock proteins (HSP) are associated with a broad array of cellular peptides that are generated during protein degradation. The HSP preparations derived from any cell (e.g. a tumour cell) contain a peptide repertoire of that cell. Vaccination with HSP peptide complexes generates T cell responses against the peptides.

### Purification of HSP70-tumour peptide complexes

B16 tumour cells (1 x10⁶) were injected into C57BL/6 mice and after 14 days mice were sacrificed and tumours removed. Tumours were homogenized in 4 times its volume of hypotonic buffer (2mM NaHCO₃, PMSF pH 7.1) then centrifuged at 100,000g and the supernatant recovered. The sample was changed to buffer D (20 mM Tris-acetate, 20 mM NaCI, 15 mM beta-mercaptoethanol, 3mM MgCI₂, 0.5 mM PMSF, pH 7.5) with a PD10 column. The sample was applied directly to an ADP-agarose column (5 ml) equilibrated with buffer D. The column was washed with buffer D containing 0.5 M NaCl and then buffer D alone until no further protein could be detected by protein assay. Finally the column was incubated with buffer D containing 3mM ADP at room temperature for 30 mins and subsequently eluted with the same buffer (25 ml). The HSP70 was quantified using a Bradford protein assay. The purity of the preparation was assessed on a silver stained gel and the specificity by western blotting with a specific antibody.

### Example 1 - CT26 tumour growth inhibits T cell responses to unrelated antigens

### Materials and Methods

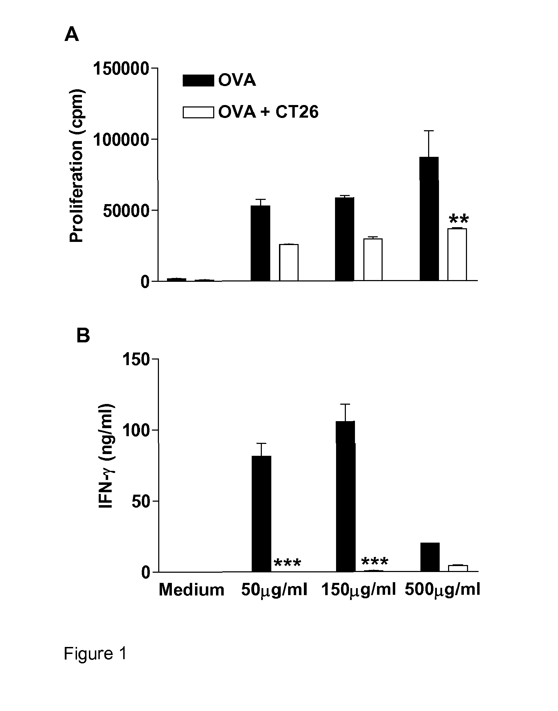

D0.11.10 mice were injected s.c. with OVA (200 µg) alone or with 2x10⁵ CT26 cells. Mice were boosted after 7 days with 200 µg OVA and 14 days later lymph node cells were re-stimulated with OVA and proliferation (Fig. 1A) was examined after 4 days and IFN-gamma (Fig. 1 B) concentrations determined in supernatants removed after 3 days. Results are mean ± SD for 5 mice per group and assayed in triplicate. OVA versus OVA + CT26,
^{**}p<0.01, ^{***}p<0.001 by ANOVA.

### Results

In order to test the possibility that the failure to generate effective adaptive immune responses during tumour growth may result from an immunosuppressive environment created by the growing tumour, we examined the influence of a growing tumour on T cell responses to unrelated antigen. OVA-TCR Tg mice were injected s.c. with OVA in the presence or absence of CT26 cells and mice were boosted with OVA after 7 days, and sacrificed 14 days later. Draining lymph node were removed and OVA-specific T cell proliferation and IFN-gamma production were tested. Mice challenged with CT26 cells and OVA had a significant reduction in OVA-specific T cell proliferation when compared with mice injected with OVA only (Fig. 1A). Co-administration of the CT26 cells also significantly reduced OVA-specific IFN-gamma production in the lymph node (Fig. 1 B), demonstrating that the growing CT26 tumour suppresses immune responses to other antigens particularly at the site of injection.

### Example 2 - Immunosuppressive cytokine production induced by growing tumours

### Materials and Methods

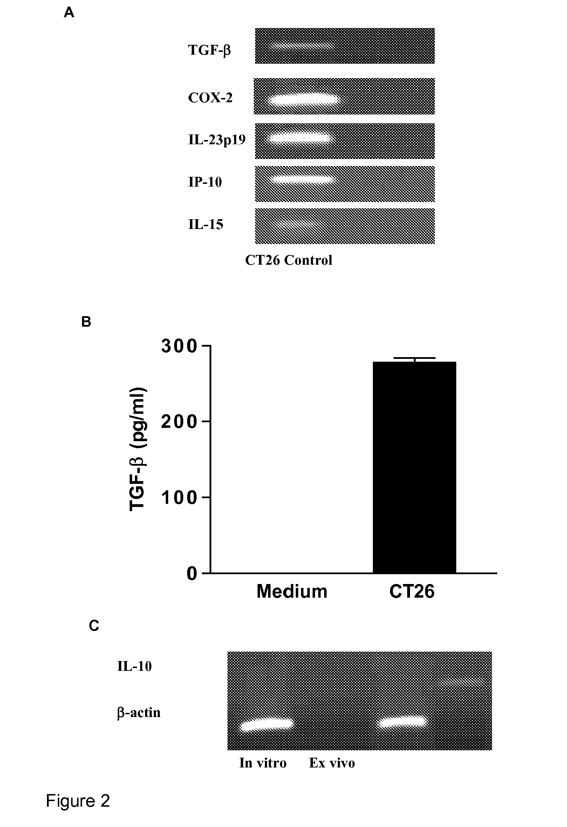

RNA was extracted from cultured CT26 cells and RT-PCR was performed using primers specific for IL-10, TGF-beta, IL-23p19, IL-15 and IP-10 (Fig. 2A). TGF-beta protein in supernatants of cultured CT26 cells was quantified by ELISA (Fig. 2B). RNA was extracted from in vitro cultured CT26 cells or homogenized solid CT26 tumours excised from mice bearing s.c. CT26 tumours (RNA pooled from 5 mice) and RT-PCR was performed using primers specific for IL-10 and beta-actin (Fig. 2C). Results are representative of 3 experiments.

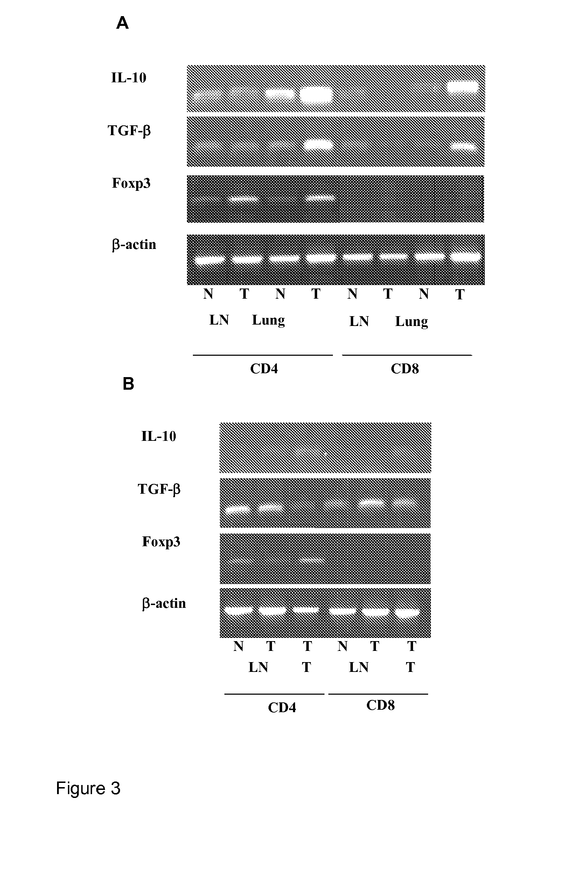

BALB/c mice (5 per group) were injected with CT26 cells either i.v. (Fig. 3A) or s.c. (Fig. 3B). Lymph nodes (LN) and s.c. tumour masses (T) or lungs were taken from naïve (N) and tumour-bearing mice (T) 14 days after tumour challenge. CD4⁺ and CD8⁺ T cells were isolated using magnetic cell sorting and RNA was isolated and RT-PCR was performed using primers specific for IL-10 and TGF-beta.

### Results

In order to examine the possible mediators of immunosuppression during tumour growth, we examined the expression of mRNA for pro- and anti-inflammatory cytokines in the cultured tumour in vitro and the growing tumour ex vivo. An examination of cytokine mRNA expression in cultured CT26 tumour cells demonstrated expression of mRNA for the anti-inflammatory cytokine, TGF-beta (Fig 2A). Furthermore, TGF-beta protein was detected in supernatants of the growing tumour (Fig 2B). Cox-2, IL-23p19, IL-15 and IP-10 mRNA was also expressed by CT26 cells (Fig 2A), but we failed to detect expression of mRNA for IL-1 beta, IL-2, IL-4, IL-5, IL-6, IL-10, IL-12p35, IL-12p40, IL-13, IL-18, IL-27p28, IL-27EB13, TNF-alpha and IFN-gamma (data not shown). Although IL-10 mRNA could not be detected in the *in vitro* cultivated tumour (Fig 2A) even after stimulation of the cells with PMA (data not shown), it was detected in the tumour mass ex vivo (Fig 2C), suggesting that cells infiltrating the tumour in vivo secreted IL-10.

We then examined IL-10 and TGF-beta mRNA expression by RT-PCR in T cells in the tumour mass, lungs and in the draining lymph nodes. CD4⁺ and CD8⁺ T cells were purified from the lungs and cervical lymph nodes of mice with CT26 lung metastases and from solid tumour and inguinal lymph nodes of mice injected s.c. with CT26 cells. Constitutive expression of IL-10 mRNA was high in CD4⁺ T cells from lungs compared with lymph nodes of naïve mice. IL-10 expression was considerably enhanced in both CD4⁺ and CD8⁺ T cells from the lungs of tumour bearing mice compared with that found in T cells from the lungs of naive mice, but there was little change in IL-10 mRNA expression in the cervical lymph nodes of mice with lung metastases (Fig 3A). In addition, TGF-beta mRNA expression was dramatically higher in both CD4⁺ and CD8⁺ T cells purified from the lungs of CT26 lung metastases bearing mice when compared with naïve control mice, with little change in the lymph nodes (Fig 3C). High IL-10 expression was also detected in both CD4⁺ and CD8⁺ T cells purified from solid tumours from mice injected s.c with CT26 (Fig 3B). There was a small increase in IL-10 mRNA expression in CD4⁺ T cells and TGF-beta mRNA expression in CD8⁺ T cells from the inguinal lymph node of mice 14 days after s.c challenge with CT26 cells (Fig. 3B). However, there was high constitutive expression of TGF-beta expression in the inguinal lymph nodes. These finding suggests that growth of CT26 tumour enhances the already immunosuppressive environment in the lung and promotes the infiltration of IL-10 and TGF-beta expressing CD4⁺ and CD8⁺ T cells to the tumour site.

### Example 3 - Foxp3 expression is enhanced in CD4⁺ T cells within the CT26 tumour mass

### Materials and Methods

BALB/c mice (5 per group) were injected with CT26 cells either intravenously (i.v.) (Fig. 3A) or subcutaneously (s.c.) (Fig. 3B). Lymph nodes (LN) and s.c. tumour masses (T) or lungs were taken from naïve (N) and tumour-bearing mice (T) 14 days after tumour challenge. CD4⁺ and CD8⁺ T cells were isolated using magnetic cell sorting and RNA was isolated and RT-PCR was performed using primers specific for Foxp3.

### Results

Having demonstrated that T cells expressing IL-10 and TGF-beta accumulate in the tumour during growth, we examined the possibility that natural Treg cells were recruited to the site of the tumour. CD4⁺ and CD8⁺ T cells were purified from inguinal lymph nodes and solid tumours in the s.c. model and superficial lymph nodes and lungs in the lung metastasis model and RT-PCR was performed using primers specific for Foxp3. In the lung metastasis model, Foxp3 expression was dramatically enhanced in CD4⁺ T cell purified from lungs and the draining lymph nodes of tumour bearing mice compared with the same tissues from naive mice (Fig 3A). Foxp3 expression was also detected in CD4⁺ T cells infiltrating the s.c. tumour mass (Fig. 3B). Unlike the lung model, Foxp3 expression was not enhanced in the draining lymph node of mice with s.c. tumours. Foxp3 expression was not detected in CD8⁺ T cells from any of the tissues from naive or tumour-bearing mice.

### Example 4 - A high frequency of IL-10-secreting CD4 and CD8 T cells and low frequency of IFN-gamma secreting T cells infiltrate the growing tumours

### Materials and Methods

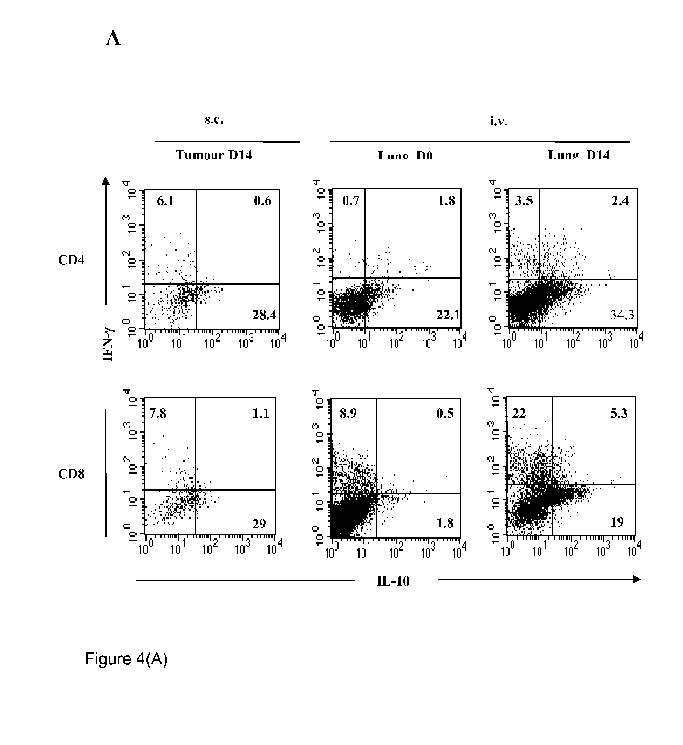

BALB/c mice were injected either s.c. or i.v. with 2x10⁵ or 3x10⁵ CT26 colon carcinoma cells respectively. Solid s.c. tumour or lungs were excised at the days indicated. Cells were stimulated with PMA and ionomycin and labelled with antibodies specific for surface CD4, CD8 and intracellular IL-10 and IFN-gamma and immunofluorescence analysis was performed (Fig. 4A).

### Results

Having demonstrated the presence of IL-10-secreting and Foxp3⁺ T cells in the growing tumour, we used intracellular cytokine staining to examine the relative frequency of effector versus regulatory T cells recruited into the lungs or tumour mass during tumour growth. Mice were challenged s.c. or i.v. with CT26 cells and tumours removed after 3 or 14 days and intracellular cytokine staining performed for IL-10 and IFN-gamma on T cells labelled for surface CD4 or CD8. A very high frequency (24-37%) of IL-10 secreting CD4⁺ and CD8⁺ T cells were detected in lungs of mice bearing CT26 metastases and in the tumour mass of mice injected s.c. with CT26 cells (Fig 4A). In contrast only 6-7 % of CD4⁺ and 9-27% of CD8⁺ T cells secreted IFN-gamma (Fig 4A).

### Example 5 - IL-10-secreting macrophages and DC infiltrate the growing tumour

### Materials and Methods

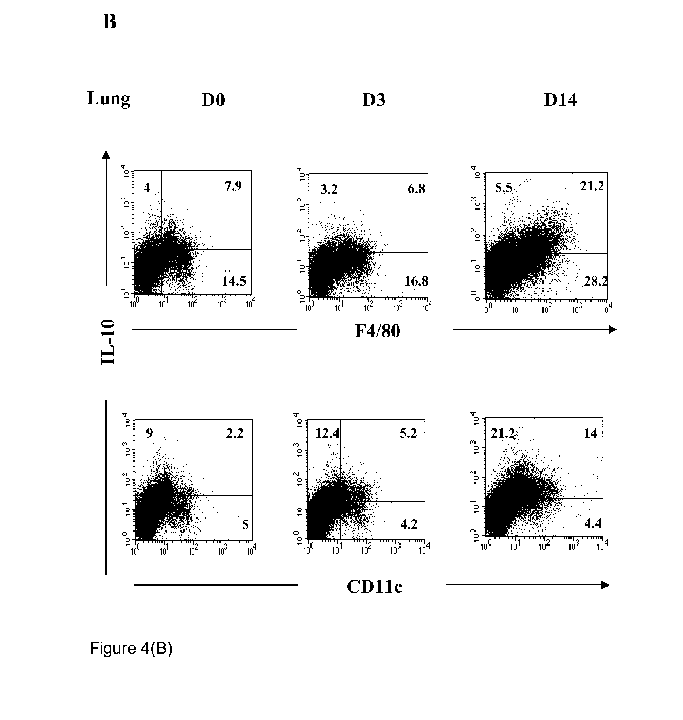

BALB/c mice were injected i.v. with 3x10⁵ CT26 colon carcinoma cells and lungs were removed from naïve mice (Day 0) or from mice 3 and 14 days post tumour challenge. Cells were labelled with antibodies specific for surface CD11 c and F4/80 and for intracellular IL-10 and immunofluoresence analysis performed (Fig. 4B).

### Results

It has been reported that innate IL-10, secreted by macrophages and DC, plays a critical role in the differentiation of inducible Treg cells from naïve T cells in the periphery. Having demonstrated recruitment of IL-10-secreting T cells to the lungs during development of CT26 metastases, we therefore examined the possibility that this may involve recruitment and activation of IL-10 producing macrophages or DC in the lungs. Mice were challenged i.v. with CT26 cells and lungs and cervical lymph nodes removed after 3 or 14 days and intracellular cytokine staining performed for cells labelled for surface CD11 c or F4/80. The development of CT26 tumours in the lungs was associated with recruitment of macrophages and DC to the lungs and a high proportion of these innate cells secreted IL-10 (Fig. 4B). The percentage of CD11 c⁺ cells in the lungs increased from 11 % in naïve mice to 17% and 34% 3 and 14 days respectively after tumour challenge, whereas the percentage of F4/80⁺ cells increased from 12% in naive mice to 27% 14 days after tumour challenge. Furthermore 79% of infiltrating macrophages and 38% of infiltrating DC secreted IL-10 on day 14 after tumour challenge. In contrast, the percentage of CD11 c+ and F4/80⁺ cells was lower in the lymph node 3 and 14 days after tumour challenge when compared with naive control mice. Furthermore, a very low frequency of these cells secreted IL-10 and this did not change significantly after tumour challenge.

### Example 6 - B16 tumours induce expression of Cox-2 in dendritic cells and macrophages

### Materials and Methods

Bone marrow derived dendritic cells or peritoneal macrophages from C57BL/6 mice were incubated with various dilutions of B16 supernatants for 24 h. Cells were homogenised, and resultant mRNA was analysed by RT-PCR using Cox-2 specific primers, and compared to beta-actin expression. B16 supernatants was prepared by growing 5 x 10⁵ B16 cells/ml for 1 week and then removing the spent media.

### Results

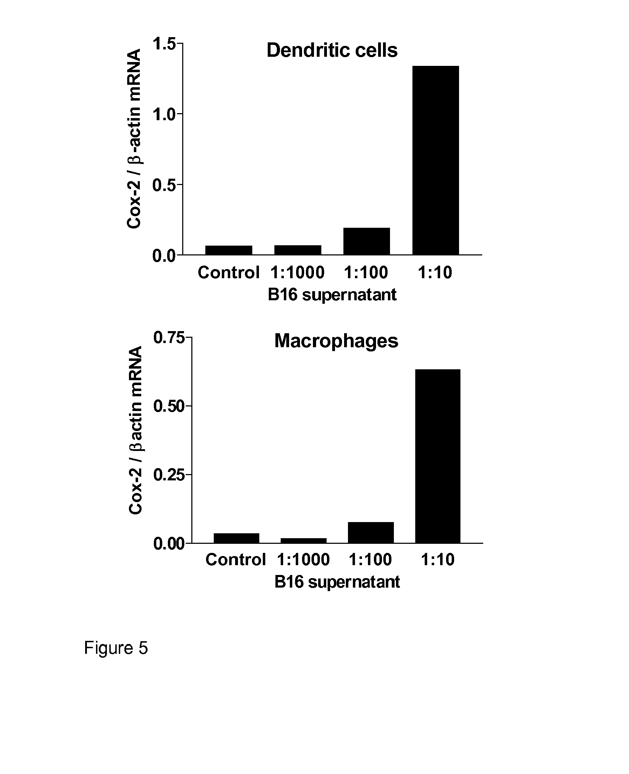

Cox-2 inhibitors are used with varying success in the treatment of cancer. Cox-2 and PGE2 induced by Cox-2 have been implicated in promoting Foxp3-expressing regulatory T cells. We have already showed that CT26 tumour expresses Cox-2 mRNA. Here we examined the possibility that tumour cell may also induce expression of Cox-2 in macrophages and dendritic cells, using the B16 tumour cell line. Stimulation of bone-marrow derived DC or peritoneal macrophages with supernatants from growing B16 cells induced Cox-2 mRNA expression in DC and macrophages (Fig. 5). Therefore tumour cells not only express Cox-2 but also induce expression of Cox-2 in cells of the innate immune system.

### Example 7 - CpG-ODN induce expression of Cox-2 in dendritic cells and macrophages

### Materials and Methods

Bone marrow-derived DC and peritoneal macrophages from C57BL/6 mice were incubated with various concentrations of CpG for 24h. Cells were homogenised, and resultant mRNA was analysed by RT-PCR using Cox-2 specific primers, and compared to beta-actin expression.

### Results

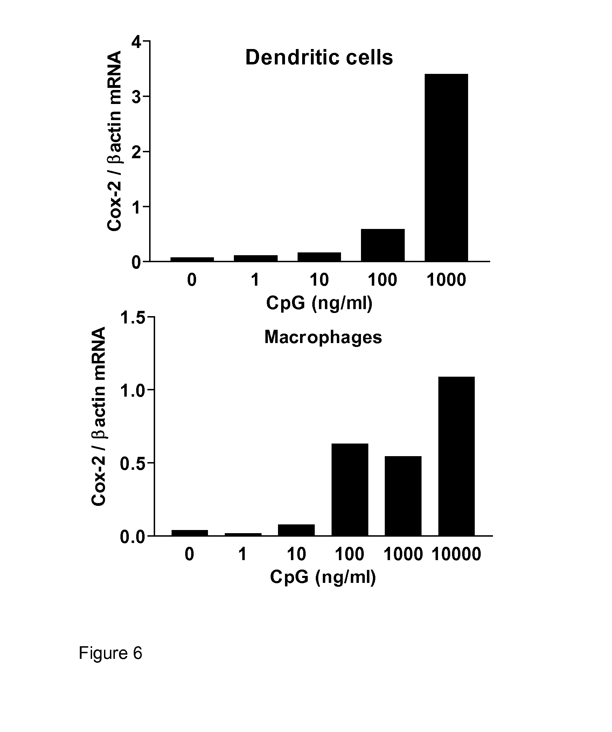

CpG-ODN are being tested as therapies for tumours and as adjuvants for infectious disease vaccines. Their potential is based on their ability to enhance innate immune responses, especially IL-12 and consequently adaptive immunity through interaction with TLR9 in innate immune cells, such as dendritic cells and macrophages. However TLR signalling is not confined to the induction of inflammatory cytokines such as IL-12 production and other mediators may also be activated. We tested the influence of CpG on Cox-2 induction in innate immune cells. CpG induced a dose dependant induction of Cox-2 mRNA expression in dendritic cells and macrophages (Fig 6). Since Cox-2 may contribute to angiogensis and anti-inflammatory responses, this is not desirable for tumour therapy or as an adjuvant for cancer or infectious disease vaccines.

### Example 8 - TLR ligands induce IL-10-secreting T cells with regulatory activity as well as IFN-gamma secreting Th1 cells

### Materials and Methods

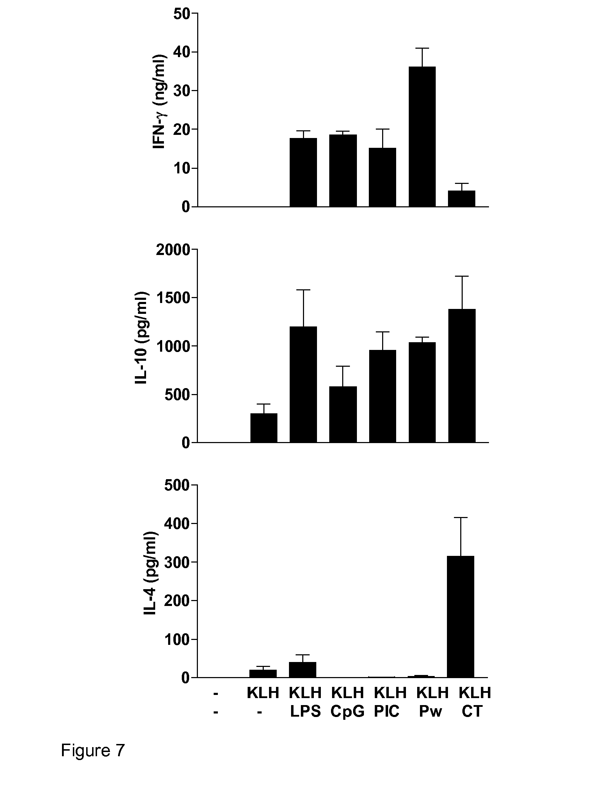

BALB/c mice were immunized s.c. with PBS only, KLH (5 µg) or KLH and CpG-ODN (25 µg), LPS (1 µg), PolylC (25 µg), killed *B. pertussis* whole cell vaccine (Pw) or CT (10 ng). After 7 days mice draining lymph node cells were stimulated with KLH (50 µg/ml) and IFN-gamma, IL-4 and IL-10 concentrations determined after 3 days by ELISA (Fig. 7).

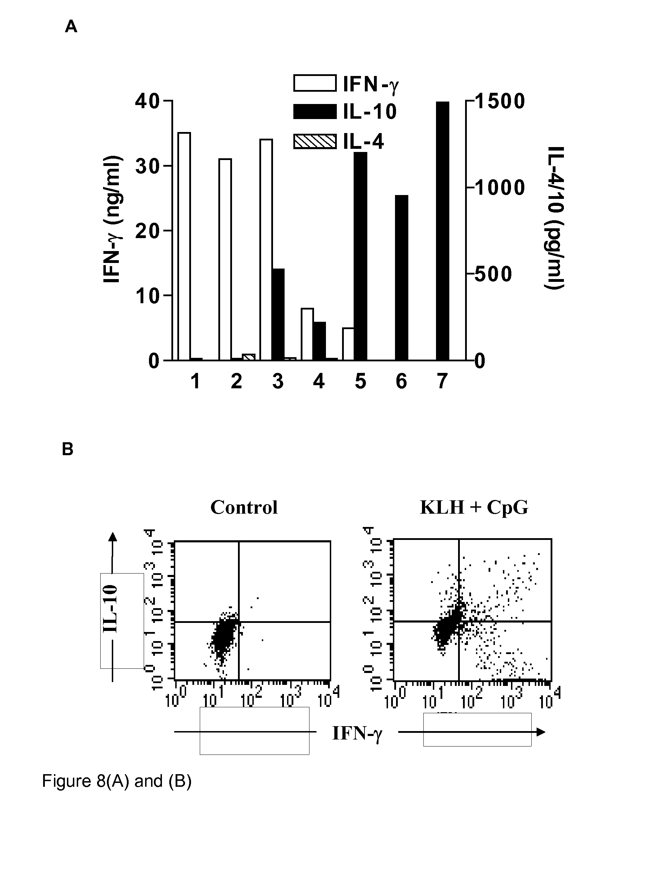

CD4⁺ T cell lines were established from mice immunized with KLH and CpG-ODN were stimulated with KLH (10 µg/ml) and APC (Fig. 8A). Lines 6 and 7 were initially stimulated with antigen in presence of anti-IFN-gamma, added at the imitation of the culture (and removed by washing at several re-culture steps) to prevent outgrowth of IFN-gamma-secreting T cells. T cell lines were tested for IFN-gamma, IL-4 and IL-10 production by stimulation with antigen and APC (Fig. 8A).

Lymph node cells from mice immunized with PBS (control) or KLH and CpG-ODN were stimulated with KLH (50 µg/ml) and after 6 days cells were re-stimulated for 6 h with PMA and ionomycin (Fig. 8B). Brefeldin A (10 µg/ml) was added for the final 4 h. Immunofluorescence analysis was performed for intracellular IL-10 and IFN-gamma after gating on CD4⁺ cells (Fig. 8B).

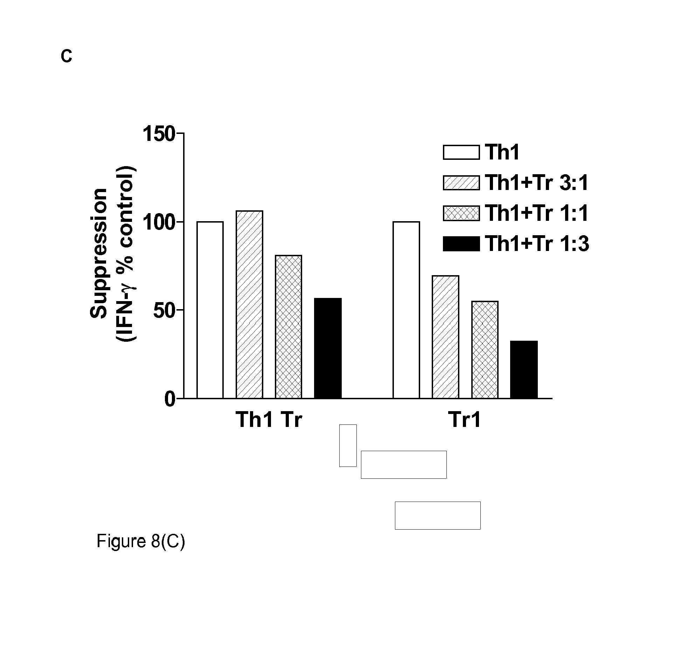

A Th1 cell line that secreted high concentrations of IFN-gamma and low or no IL-4 and IL-10 was established from mice immunized with KLH and CpG-ODN by culturing T cells in the presence of IL-12 and anti-IL-10 (Fig. 8C). A Tr1-type cell line, that secreted IL-10 and low or no IFN-gamma or IL-4 was established by initial culture in the presence of anti-IFN-gamma and a mixed IFN-gamma and IL-10 secreting T cell line (termed Th1 Tr) was established by culturing with antigen and APC without the addition of cytokines or antibodies. The Th1 cell line was culture with APC and antigen alone or in the presence of Tr1 or Th1 Tr cells at ratio of 1:3. 1:1 or 3:1. Supernatants were removed after 3 days and the concentration of

IFN-gamma was tested by ELISA (Fig. 8C). Results are expressed relative to the response of the Th1 cell alone

### Results

It has been extensively reported that TLR ligands, such as CpG or LPS selectively induce Th1 responses or, in the case of certain TLR-2 agonists, Th2 responses. We examined the role of TLR ligands in directing T cell responses to a model bystander antigen, KLH. Immunization with KLH alone generated T cells that secrete IL-10 and low concentrations of IL-4 but no IFN-gamma. Co-administration with the TLR ligands, CpG-ODN, LPS or PolylC, generated T cells that secreted high concentrations of IFN-gamma, but low or undetectable IL-4, a cytokine profile consistent with the induction of Th1 cells (Fig. 7).

However, in addition to IFN-gamma, significant IL-10 (but not IL-4) was also detected in supernatants from antigen-stimulated lymph node cells from mice immunized with KLH in the presence of TLR ligands (Fig. 6). Killed *B. pertussis* and pertussis whole cell vaccines have potent adjuvant activity. Here we found that killed *B. pertussis* promoted the induction of antigen-specific T cells that secreted IL-10 and IFN-gamma to a co-administered antigen, KLH. In contrast, immunization with CT, which we had previously shown induces Tr1 and Th2 cells, enhanced KLH-specific IL-4 and IL-10 production.

The generation of KLH-specific CD4⁺ T cells lines from immunized mice showed that immunization with KLH and CpG-ODN (Fig 8A) generated T cells that secreted IFN-gamma only or IFN-gamma and IL-10. When T cell lines generated from mice immunized with antigen and CpG-ODN or *B*. *pertussis* were generated by initially culturing ex vivo lymphocytes in the presence of anti-IFN-gamma (which was then removed by several washing steps and several re-culturing steps), these T cells secreted IL-10, but not IFN-gamma or IL-4 (Fig 8A). These findings suggest that Th1-promoting adjuvants also generate IL-10 secreting Tr1-type T cells and T cells that secrete IL-10 and IFN-gamma, which have been termed Th1Tr cells. Intracellular cytokine staining on CD4⁺ T cells from mice immunized with KLH and CpG-ODN also revealed distinct populations of cells that secreted IFN-gamma or IL-10 alone or IFN-gamma and IL-10 (Fig. 8B), confirming that this TLR agonist promotes the induction of Th1 and Tr1 cells and a distinct population that secretes both cytokines.

We next examined the suppressor function of the antigen-specific IL-10 or IL-10 and IFN-gamma secreting T cells induced by immunization with KLH and CpG-ODN against an established KLH-specific CD4⁺ Th1 cell line. This Th1 cell line was established from a mouse immunized with KLH and CpG-ODN and secretes high concentrations of IFN-gamma in response to stimulation with antigen and APC. Tr1 cells that secreted IL-10, but not IFN-gamma or IL-4 were also expanded from spleen cells of mice immunized with KLH and CpG-ODN by initial culture in the presence of a neutralizing anti-IFN-gamma antibody. These Tr1 cells significantly suppressed IFN-gamma production by Th1 cells at a ratio of 3:1, 1:1 and 1:3, with the greatest expression observed with the highest number of Tr1 cells (Fig 8C). The Th1 Tr cells, which secreted IFN-gamma and IL-10 also suppressed IFN-gamma secretion, but only at ratios of 1:1 and 3:1. These findings suggest that in addition to inducing effector IFN-gamma secreting cells, CpG-ODN simultaneously promote the induction of T cells with suppressor activity.

### Example 9 - TLR ligands induce DC IL-10 production through activation of ERK and p38

### Materials and Methods

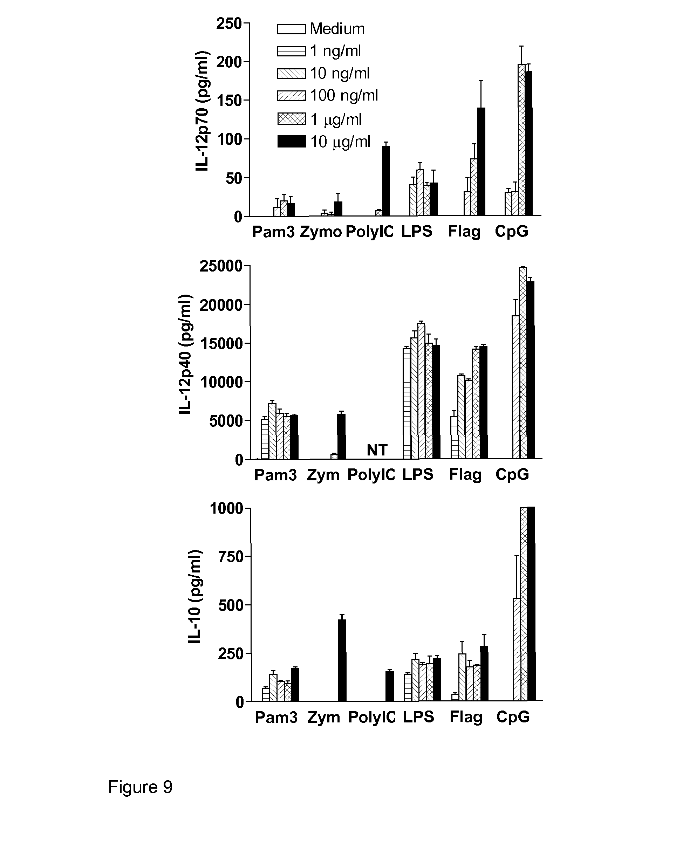

DC were incubated with 1 ng/ml -10 µg/ml Pam3CSK4 (Pam3), Zymosan (Zym), PolylC, LPS, Flagellin (Flag), CpG-ODN or medium only. After 24 h, supernatants were removed and IL-10, IL-12p40 and IL-12p70 concentrations determined by ELISA. NT, not tested (Fig. 9).

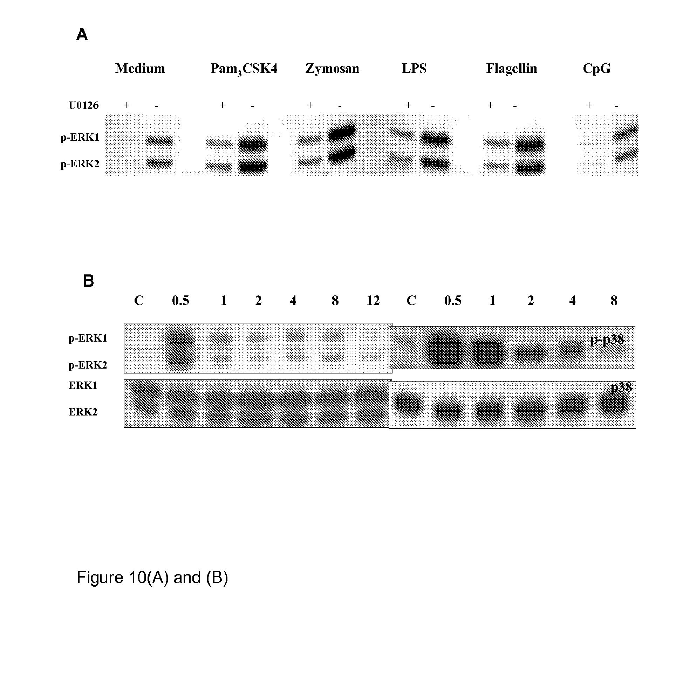

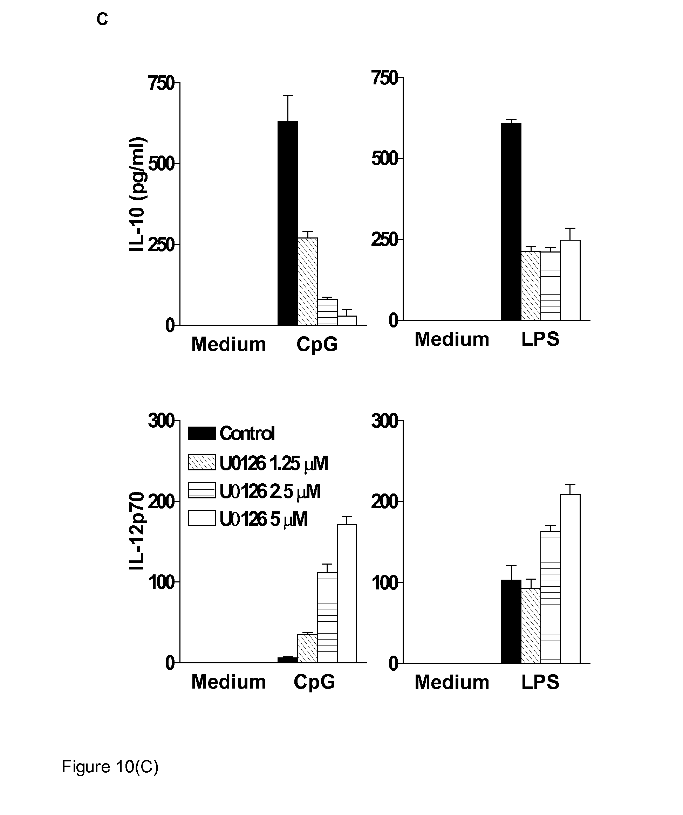

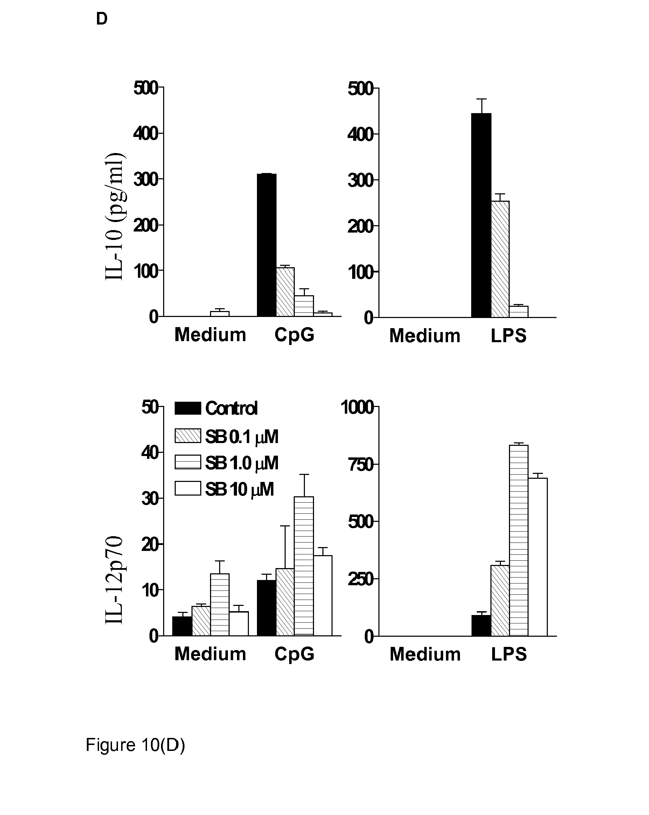

DC were pre-incubated for 1 h with the MEK 1/ 2 inhibitor (U0126) and then incubated for 15 min with medium only or with Pam3Cys, Zymosan, LPS, Flagellin, or CpG-ODN (1 or 5 µg/ml). Cells were lysed and Western blots performed using antibodies specific for pERK1 and pERK2 (Fig. 10A). DC were stimulated with medium only (0) or for 0.5, 1, 2, 4, 8 or 12 h with LPS (100 ng/ml). Western blotting was performed using antibodies specific for pERK or p38 (Fig. 10B). Blots were stripped and re-probed with antibodies specific for total ERK or p38. DC were pre-incubated with medium only (control), U0126 (1.25-5 µM) (Fig. 10C) or the p38 inhibitor, SB203580 (SB; 0.1 - 10 µM) (Fig. 10D) for 1 h before the addition of CpG-ODN (5 µg/ml), LPS (100 ng/ml) or medium only and 24 h later IL-10 concentrations were determined by ELISA. Cells treated with DMSO were used as negative controls.

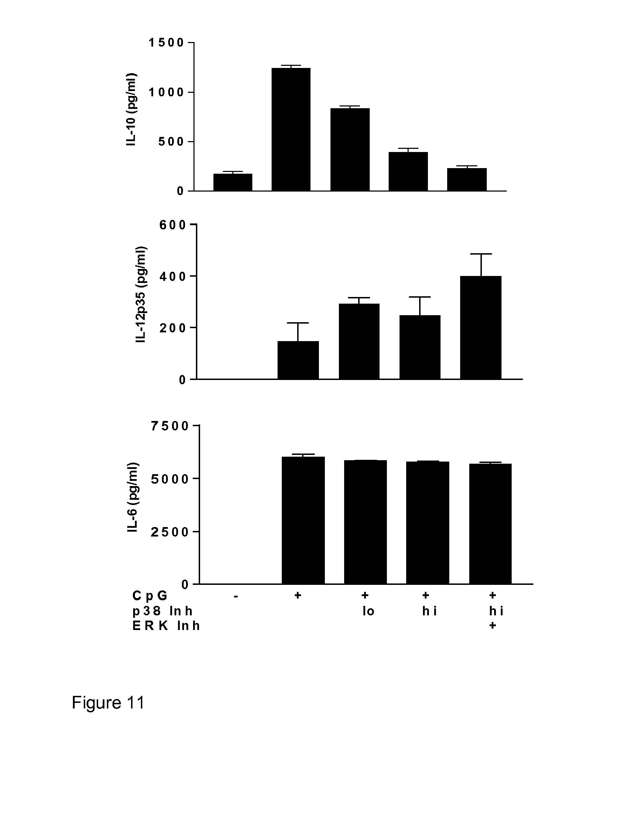

Bone marrow-derived DC from BALB/c mice were incubated with CpG (10 µg) alone or with the p38 inhibitor SB203580 (lo:1µM, hi:5 µM), or with the ERK inhibitor U0126 (5 µM) for 24 hours. Superntants were removed, and aliquots was measured for IL-10, IL-12p70 and IL-6 by ELISA (Fig. 11 ).

### Results

Having demonstrated that TLR ligands, and killed *Bordetella pertussis,* that includes TLR ligands, induce Tr cells as well as Th1 cells, we examined the possibility that this may reflect simultaneous induction of IL-10 and IL-12 by DC. We found that each of the TLR ligands examined, Pam3CSK4 (TLR-2), Zymosan (TLR-2), PolylC (TLR-3), LPS (TLR-4), Flagellin (TLR-5) and CpG-ODN (TLR-9) induced production of IL-10 as well as IL-12p40 and IL-12p70 from immature bone marrow-derived DC (Fig. 9). The induction of IL-10 and IL-12 has been linked to ERK and p38 signalling respectively; therefore we examined the role of these MAP kinases in TLR-induced cytokine production. We found that each of the TLR ligands examined induced ERK phosphorylation in DC (Fig. 10A). LPS-induced ERK phosphorylation was maximal at 30 minutes but was still evident for up to 8 hours (Fig. 10B). Pre-incubation with the MEK 1/2 inhibitor, U0126, which inhibited TLR-ligand induced ERK phosphorylation (Fig. 10A), suppressed IL-10 production from CpG-ODN- or LPS-stimulated DC (Fig. 10C). The MEK 1/2 inhibitor enhanced IL-12p70 production and this effect was retained in DC from IL-10^{-/-} mice, suggesting that it was not secondary to a reduction in IL-10 production (data not shown). The TLR ligands also induced phosphorylation of p38 in DC. Pre-incubation of DC with the specific p38 inhibitor, SB203580, inhibited IL-10 and enhanced or IL-12p70 production in response to LPS or CpG-ODN (Fig 10D and Fig 11 ). We also found that the combination of the p38 and ERK inhibitors further suppressed CpG-induced IL-10 production and enhanced IL-12p35, but had no effect on IL-6 (Fig 11). It has previously been suggested that ERK and p38 may differentially regulate IL-10 and IL-12 production, with ERK promoting IL-10 and p38 promoting IL-12. However our findings, combined with recent reports using IRF-5-defective mice (Takaoka, A., H. Yanai, S. Kondo, G. Duncan, H. Negishi, T. Mizutani, S.I. Kano, K. Honda, Y. Ohba, T.W. Mak, and T. Taniguchi. 2005. Integral role of IRF-5 in the gene induction programme activated by Toll-like receptors. Nature*)* and a NF-kappaB inhibitor (Kabashima, K., T. Honda, Y. Nunokawa, and Y. Miyachi. 2004. A new NF-kappaB inhibitor attenuates a TH1 type immune response in a murine model. FEBS Lett 578:36-40), suggest that TLR ligands activate IL-10 through the MAP kinases, ERK and p38, while IL-12p70 utilizes the IRF-5 and NF-kappaB pathways.

### Example 10 - Addition of p38, ERK and Cox-2 inhibitors to dendritic cells stimulated with foreign antigen and CpG enhance their ability to induce Th1 and suppress the induction of IL-10-secreting T cells

### Materials and Methods

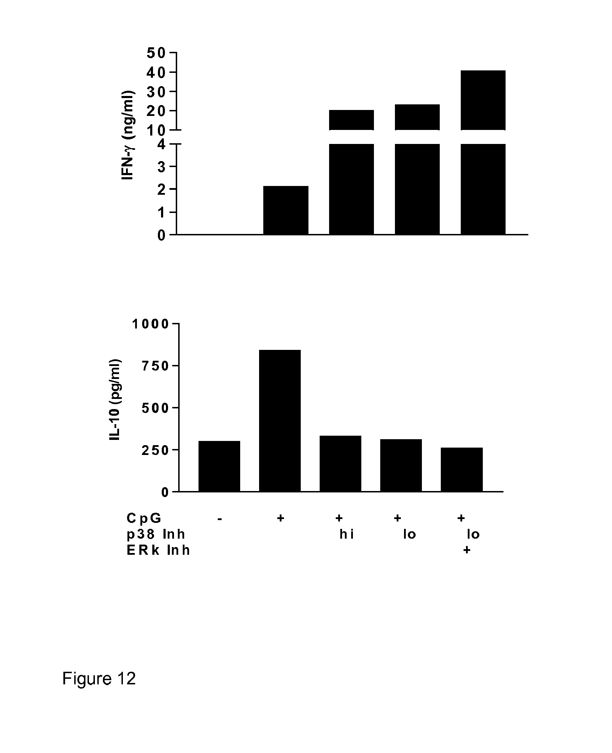

Bone marrow-derived DC from BALB/c were incubated with OVA Class II peptide (5 µg). Concurrently, cells were stimulated with either CpG (10 µg) alone or with the p38 inhibitor SB203580 (lo:1µM, hi:5 µM) or P38 inhibitor and an ERK inhibitor U0126 (5 µM) for 24 hours. Purified CD4+ T cells from D011.10 OVA-TCR Tg mice were stimulated with the DC (10:1) and supernatants were removed 3 days after antigen stimulation and IL-10 and IFNgamma quantified by ELISA (Fig. 12).

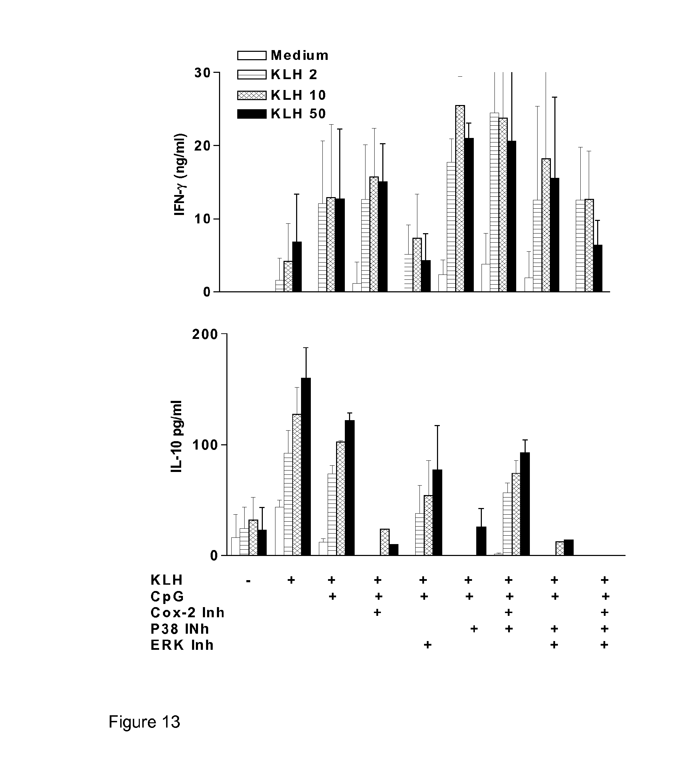

Bone marrow-derived DC from C57BL/6 mice were incubated with medium only (-), KLH (5 µg) and CpG (5 µg) and the indicated combinations of inhibitors to Cox-2 (NS-398: 5 µM), p38 (SB203580: 1 µM), ERK (U0126: 5 µM). 2.5 x 10⁵ treated cells were injected into each footpad of 57BL-6 mice. Popliteal lymph nodes and spleens were removed after 5 days, and single cell suspensions were restimulated with KLH (2, 10, 50 µg/ml). Supernatants were removed after 3 days, and IFN-gamma and IL-10 concentrations quantified by ELISA (Fig. 13).

### Results

TLR ligands enhance IL-10 and IL-12 production from dendritic cells and macrophages. Furthermore, IL-12 and IL-10 production from innate immune cells promote the induction of Th1 and Treg cells respectively. This explains the simultaneous induction of Th1 and Treg cells with TLR agonists as adjuvants. Here we show that inclusion of p38 or ERK inhibitors or a combination of both with CpG enhances Th1 and suppresses Tr induction to a foreign antigen in vitro and in vivo. DC were stimulated with OVA peptide alone or with CpG or with CpG in the presence of p38 inhibitor or P38 and ERK inhibitors and then used to stimulate CD4⁺ T cells purified from spleen of D011.10 OVA- T cell receptor (TCR) transgenic (Tg) mice. T cell cytokine production was examined after one or two rounds of antigen stimulation. The results show that DC pulsed with OVA in the presence of CpG induced IL-10 and IFN-gamma-secreting T cells, which is consistent with the in vivo induction of Th1 and Treg cells (Fig 12). Addition of the p38 inhibitor suppressed the induction of IL-10 secreting T cells and enhanced IFN-gamma-secreting T cells. This was further enhanced by the addition of the ERK inhibitor.

We next examined the influence of p38, ERK and Cox-2 inhibitors on the ability of CpG-activated dendritic cells to induce T cell responses in vivo. Bone marrow derived DC were stimulated with KLH alone or with CpG alone or with combination of p38, ERK and Cox-2 inhibitors and then injected s.c into mice. The draining lymph nodes and spleen were removed 5 days later and cells stimulated with antigen (KLH) and cytokine production was assessed. The data revealed that DC pulsed with antigen in the presence of CpG induced IL-10 and IFN-gamma-secreting T cells in vivo (Fig 13). Addition of the p38 or Cox-2 inhibitors to the DC during stimulation with CpG and antigen prior to injection into the mice enhanced the ability of the DC to induce IFN-gamma-secreting T cells, while suppressing IL-10 secreting T cells. The ERK inhibitor suppressed induction of IL-10-secreting T cells, but also suppressed Th1 cells. The combination of ERK, p38 and Cox-2 inhibitor completely suppressed IL-10 producing T cells, but also reduced IFN-gamma-secreting T cells. These finding demonstrate that p38 and Cox-2 inhibitors enhance Th1 and suppress Treg cells induction to foreign antigens with a TLR agonist as the adjuvant. The ERK inhibitor also suppressed IL-10-producing T cells.

### Example 11 - Cox-2 inhibitors suppress TLR agonist-induced IL-10 and TGF-beta production and enhance innate IL-12 and IL-27 production

### Material and Methods

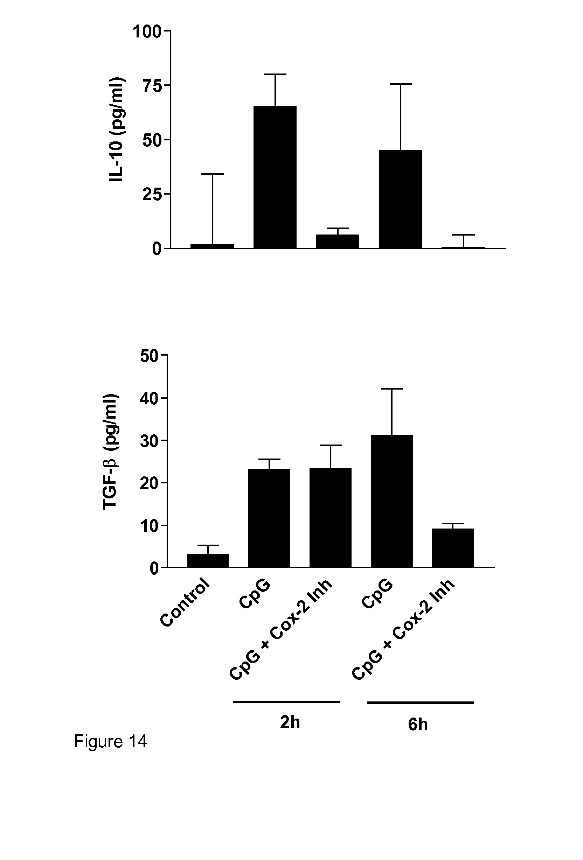

C57BL/6 mice were injected into the flank with either CpG (10 µg) or with CpG and the Cox-2 inhibitor Celecoxib (100 µg). The draining inguinal lymph nodes were removed after 2 and 6 h, passed through a sieve and resuspended in 1 ml of PBS. Cells were removed, and IL-10 and TGF-beta concentrations in the supernatants quantified by ELISA (Fig. 14).

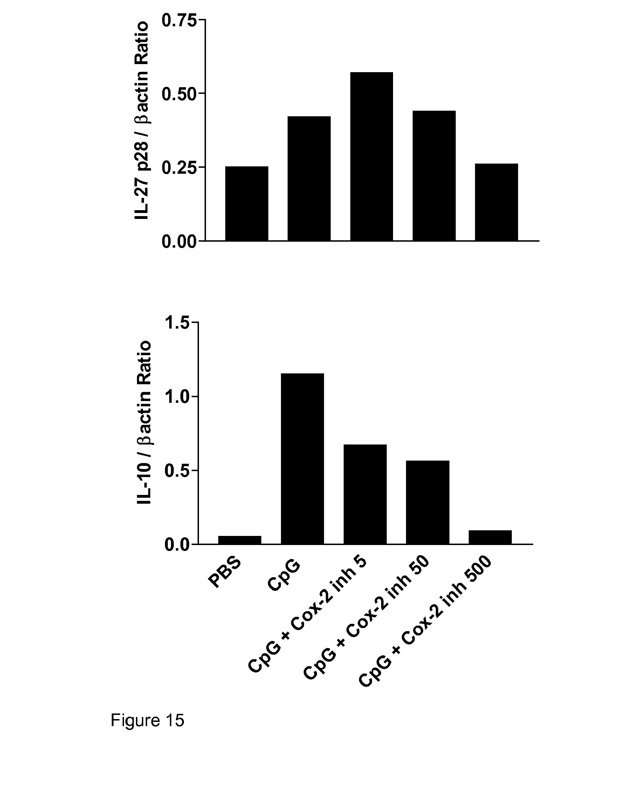

C57BL/6 mice were injected into the flank with either CpG (10 µg) alone or with the various concentrations of the Cox-2 inhibitor Celecoxib (5, 50 or 500 µg). After 6 h the draining inguinal lymph nodes were removed and single cell suspensions prepared. Cells were homogenised, and resultant mRNA was analysed for expression of IL-27 and IL-10 by RT-PCR, and compared to beta-actin expression (Fig. 15).

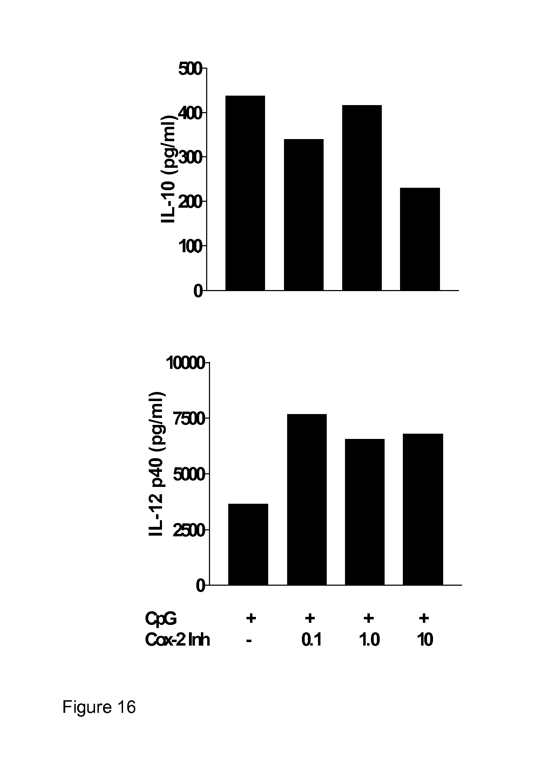

Bone marrow-derived DC from C57BL/6 mice were incubated with CpG (10 µg) with various concentrations of the Cox-2 inhibitor NS-398 for 24 h. Supernatants were removed and IL-10 and IL-12p40 concentrations determined by ELISA (Fig. 16).

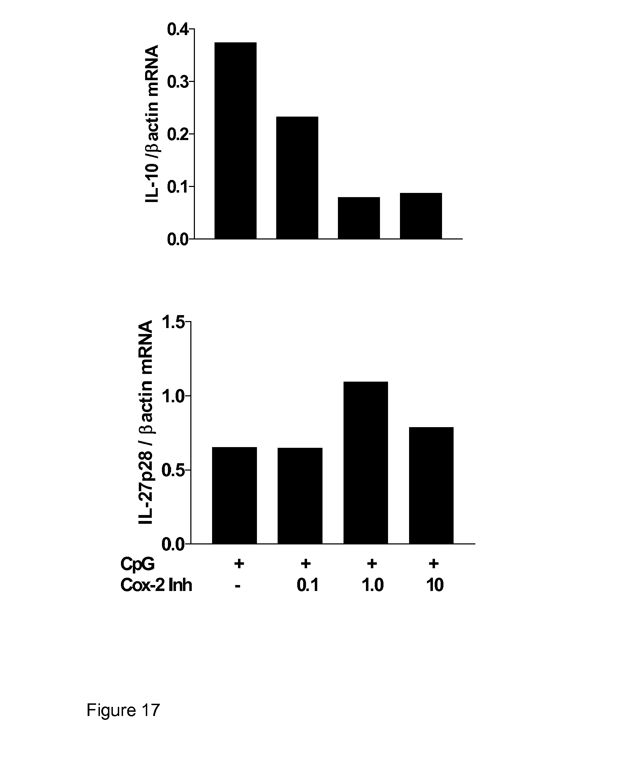

Bone marrow-derived DC from C57BL/6 mice were incubated with CpG (10 µg) with various concentrations of the Cox-2 inhibitor NS-398 for 24 h. Cells were homogenised, and resultant mRNA was analysed by RT-PCR using IL-10 and IL-27p38 specific primers, and compared to beta-actin expression (Fig. 17).

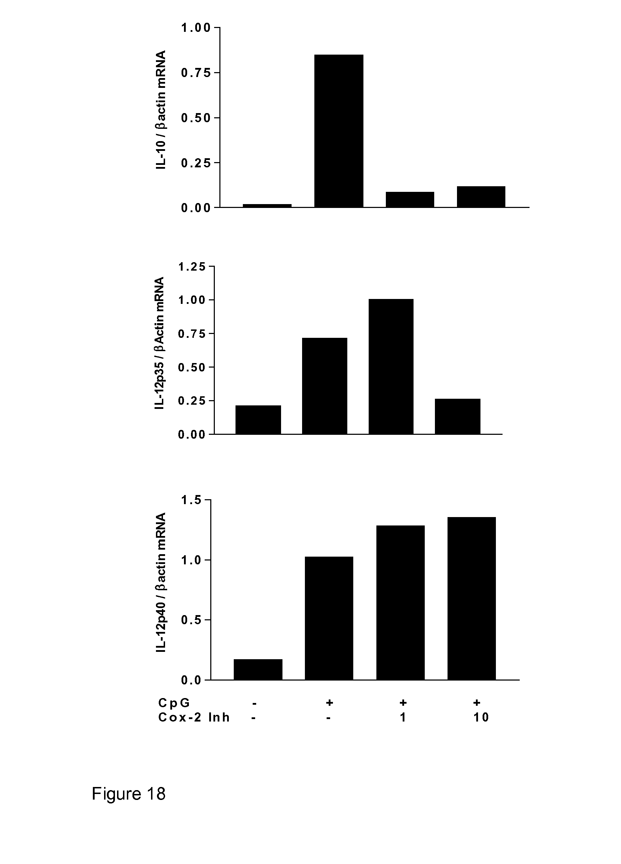

Bone marrow-derived DC from C57BL/6 mice were incubated with CpG (10 µg) or with 1 or 10 µM of the Cox-2 inhibitor NS-398 for 24 h. Cells were homogenised, and resultant mRNA was analysed by RT-PCR using IL-10, IL-12p35 or IL-12p40 specific primers, and compared to beta-actin expression (Fig. 18).

### Results

Since we found that tumour cells express Cox-2 and tumour supernatants and CpG induce Cox-2 expression in innate immune cells, we examined the influence of a Cox-2 inhibitor on TLR agonist-induced cytokine expression in vivo and in vitro from dendritic cells. Mice were injected with s.c. with CpG alone or in the presence of Cox-2 inhibitor and lymph nodes were removed 6 hours later and cytokine production assessed. Injection of CpG induced production of IL-10 and TGF-beta, which was suppressed by co-administration of the Cox-2 inhibitor (Fig 14). CpG-induced expression of IL-10 mRNA was also suppressed in mice by co-administration of the Cox-2 inhibitor, whereas expression of IL-27 mRNA was enhanced (Fig 15). In vitro stimulation of DC with CpG induced production of IL-10 and IL-12p40. Addition of a Cox-2 inhibitor to the culture suppressed IL-10 and enhanced IL-12 production (Fig 16). CpG also induced expression of IL-12p40, IL-12p35 and IL-27p28 mRNA, which was enhanced by addition of the Cox-2 inhibitor (Fig 17 and 18). In contrast, CpG-induced IL-10 mRNA expression was suppressed by the Cox-2 inhibitor. These observations demonstrate that two distinct Cox-2 inhibitors have the ability to suppress anti-inflammatory and Treg promoting cytokines while enhancing regulatory cytokines that direct the induction of Th1 cells. Therefore the combination of TLR agonist and Cox-2 inhibitor is an effective method for priming innate and adaptive effector responses against foreign and tumour antigens in vivo.

### Example 12 - Therapy with a combination of Cox-2 inhibitor and CpG suppresses tumour growth

### Material and Methods

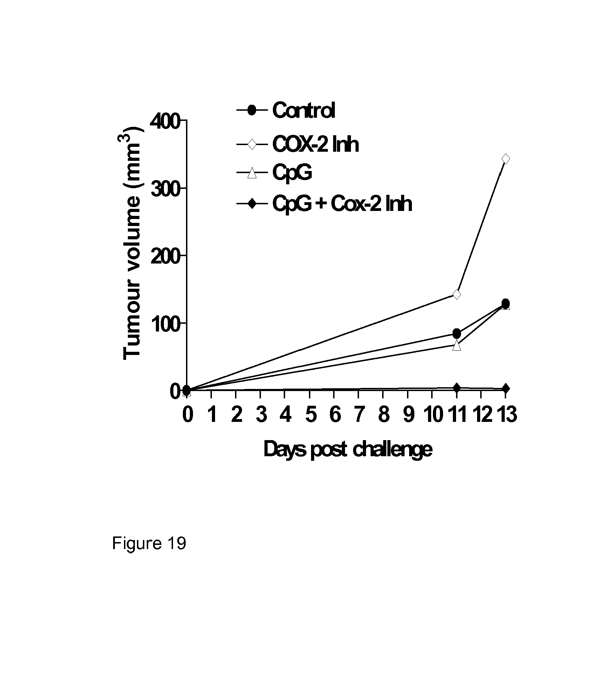

C57BL/6 mice were challenged with 2 x 10⁵ B16 tumour cells s.c. and injected s.c. on days 3, 5 and 7 with either CpG (10 µg), the Cox-2 inhibitor Celecoxib (100 µg) or a combination of both at the tumour site. Mice were routinely monitored for tumour growth (Fig. 19).

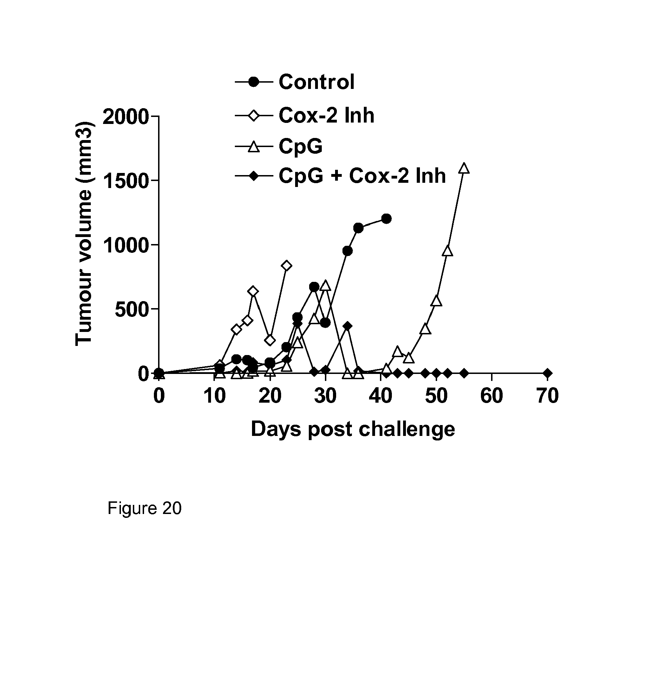

C57BL/6 mice were challenged with 2 x 10⁵ B16 tumour cells s.c. and injected s.c. on days 3, 5 and 7 with either CpG (20 µg), the Cox-2 inhibitor Celecoxib (500 µg) or a combination of both at the tumour site. Tumour volumes were routinely monitored (Fig. 20).

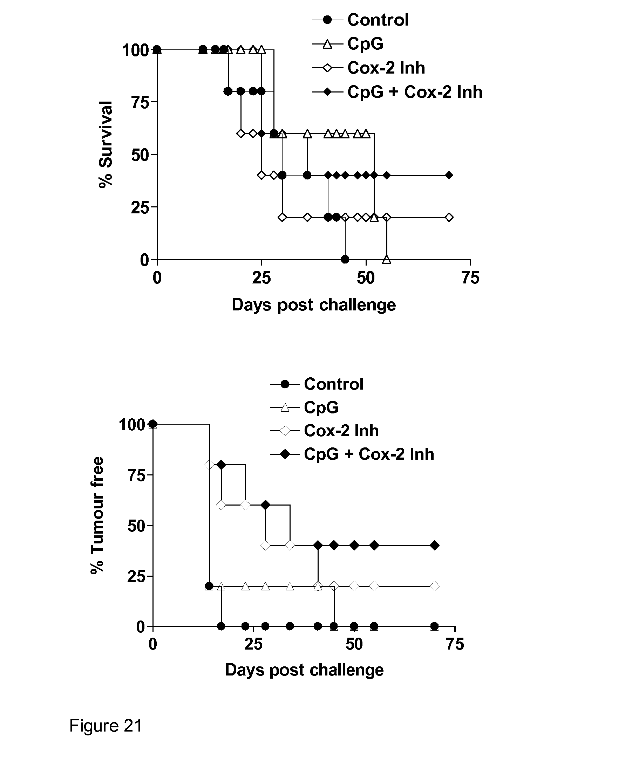

C57BL/6 mice were challenged with 2 x 10⁵ tumour cells s.c. and injected s.c. on days 3, 5 and 7 with either CpG (20 µg), the Cox-2 inhibitor Celecoxib (500 µg) or a combination of both at the tumour site. Mice were routinely monitored for tumours and survival (Fig. 21).

### Results

We used a murine tumour model to assess the efficacy of therapy with a TLR agonist combined with Cox-2 inhibitor. Mice were challenged s.c. with B16 tumour and either left untreated or treated on days 3, 5 and 7 with CpG (10 ug) injected s.c into the tumour site with and without a Cox-2 inhibitor (Celecoxib 100 ug), or with the Cox-2 inhibitor only. Treatment with the Cox-2 inhibitor alone enhanced tumour growth and CpG alone had little effect (Fig 19). However the combination of CpG and Cox-2 inhibitor resulted in a profound reduction in tumour growth. These studies were repeated using higher doses of the CpG (20 ug) and the Cox-2 inhibitor (500 ug). The higher dose of CpG did reduce tumour growth, but the protective efficacy was significantly improved when combined with therapy with the Cox-2 inhibitor (Fig 20). The protective effect of the CpG and Cox-2 inhibitor over CpG alone or Cox-2 inhibitor alone was also clearly evident from the survival curves and number of tumour free mice (Fig 21 ). All untreated mice died within 45 days and all mice treated with CpG only died within 55 days. 20% of mice treated with the Cox-2 inhibitor survived, whereas 40% mice treated with the CpG and the Cox-2 inhibitor survived.

### Example 13 - Cox-2 or ERK inhibitors enhance the therapeutic efficacy of dendritic cell vaccination against tumours

### Material and Methods

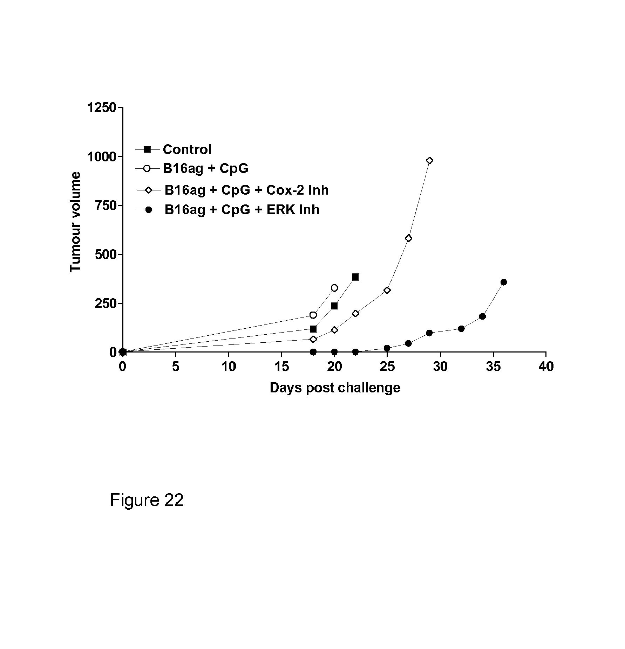

C57BL/6 mice were challenged with 2 x 10⁵ B16 tumour cells s.c. and treated with 3 injections s.c. of treated DC (1-5 x 10⁵) one week apart, starting on day 3 into the tumour site. DC were pulsed with heat shocked / irradiated B16 tumour cells and CpG (5 µg/ml) alone or with either the Cox-2 inhibitor Celecoxib (5 µM) or the ERK inhibitor UO126 (5 µM) for 24h. Mice were routinely monitored for tumour growth (Fig. 22).

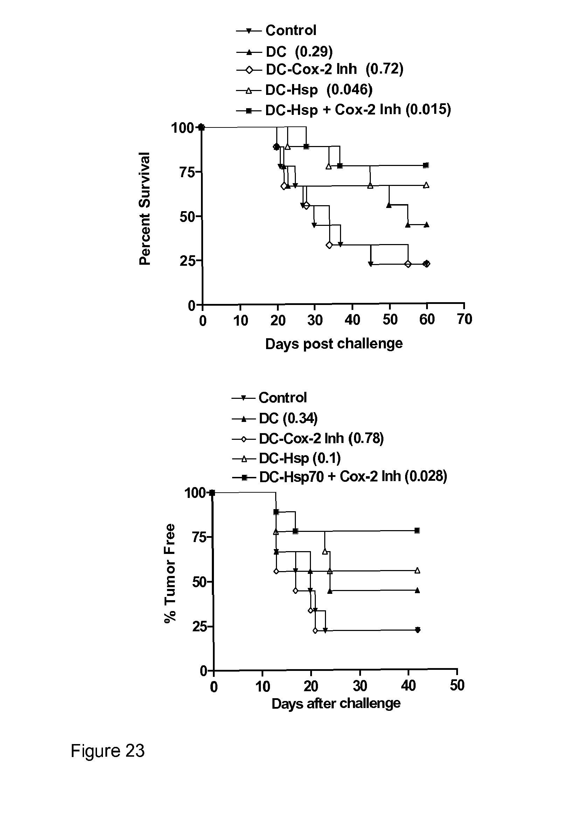

Groups of 11 C57BL/6 mice were challenged with 2 x 10⁴ B16 tumour cells s.c. and treated with 3 injections (s.c. into the tumour site) of treated DC (1-5 x 10⁵) 3, 10 and 17 days after tumour challenge. DC were incubated with HSP-70 (5 µg/ml) either alone or with the Cox-2 inhibitor NS398 (5 µM) for 24h. Mice were routinely monitored for tumour growth and survival. Figures in brackets refer to significance values in log rank analyses (Fig. 23).

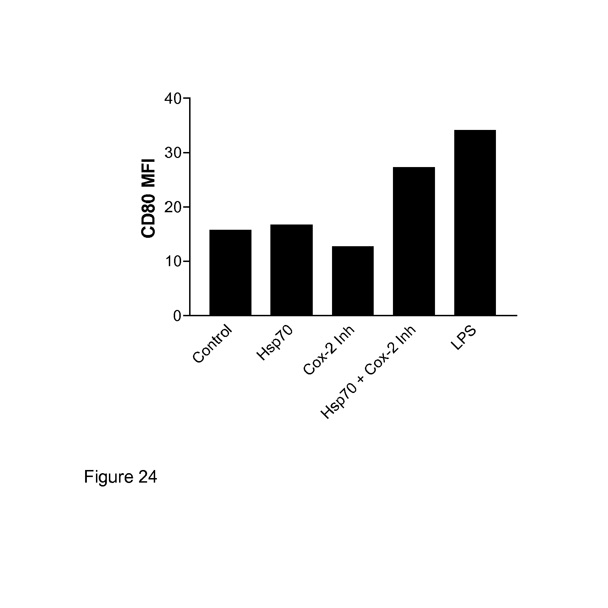

Bone marrow derived DC were incubated with medium only (Control), HSP70 vaccine (5 mg/ml), the Cox-2 inhibitor NS398 (5 mM),HSP70 and Cox-2 inhibitor or LPS (10 ng/ml). After 24 hours cells were labelled with anti-CD80 and CD11 C antibodies and cytofluorometric analysis performed. Results represent mean fluorescence intensity (MFI) on cells that were gated on the CD11 c+ DC population (Fig. 24).

### Results

In addition to their potential therapeutic efficacy in cancer, TLR agonists have adjuvant properties and enhance immune responses induced with pathogen or tumour antigens. It has been shown that the TLR agonist CpG can enhance IL-10 production from innate cells and as a consequence induce Treg cells, which may be counterproductive in cancer or infectious disease vaccines. The present inventors have surprisingly shown that they can improve the efficacy of tumour vaccines by co-administration of Cox-2 or ERK inhibitors. Rather than using direct immunization with tumour antigen and adjuvants, which will be influenced by the immunosuppressive environment of the tumour (as shown in Fig 1 ), our approach was to use dendritic cells pulsed with antigens in the presence CpG with and without inhibitors. We used heat shocked and irradiated B16 cells as a whole cell tumour vaccine, which were incubated with DC and CpG in the presence or absence of a Cox-2 or ERK inhibitor. Therapeutic immunization with dendritic cells pulsed with tumour antigens in the presence of CpG did not affect tumour growth (Fig 22). However immunization with DC pulsed with B16 antigen and CpG in the presence of the Cox-2 or ERK inhibitor significantly slowed the rate of tumour growth.

We also examined the influence of the Cox-2 inhibitor on the efficacy of therapeutic immunization with a heat-shock protein (HSP)-70 vaccine. This HSP-70 vaccine prepared by purification from B16 tumours, has previously been shown to be effective for prophylactic immunization against B16 tumours in mice, but has little effect when administered therapeutically to mice with growing tumours. Here we pulsed dendritic cells with HSP-70 vaccine in the presence or absence of the Cox-2 inhibitor and then transferred the cells 3, 10 and 17 days after B16 tumour challenge. Administration of DC pulsed with the Cox-2 inhibitor alone did had no protective effect (Fig 23). Furthermore, immunization with DC pulsed with HSP-70 vaccine alone did not enhance survival or the number of tumour free mice over that seen with by administration of un-pulsed DC. However the greatest number of tumour free animals (77%) and highest percent survival (77%) was observed in mice immunized therapeutically with DC pulsed with HSP-70 vaccine in the presence of the Cox-2 inhibitor. These finding demonstrate that the combination of Cox-2 inhibitor and HSP-70 vaccine have a surprising improved efficacy in cancer therapy over either treatment alone.

An examination of the effect of the Cox-2 and HSP70 on DC activation revealed that neither Cox-2 nor HSP70 alone enhanced CD80 expression, whereas the combination significantly enhanced CD80 mean fluorescence intensity to levels comparable to that achieved with LPS (Fig. 24). These finding suggest that the combination of HSP70 and Cox-2 inhibitor matures dendritic cells, an effect that was not observed with either molecule alone.

### Example 14 - Inhibition of ERK, Cox-2 or p38 suppresses TLR agonist induced PGE2 in dendritic cells

### Material and Methods

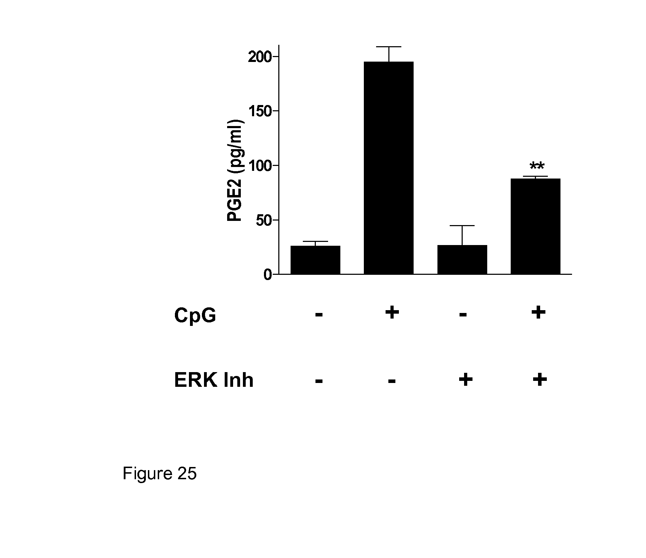

Bone marrow-derived dendritic cells (DC) were stimulated *in vitro* with CpG (5 µg) or the ERK inhibitor, U0126 (5µM), or both for 24 hours. Supernatants were removed and PGE₂ concentrations determined by ELISA. ^{**} p<0.01 CpG + p38 inhibitor versus CpG by ANOVA (Fig. 25).

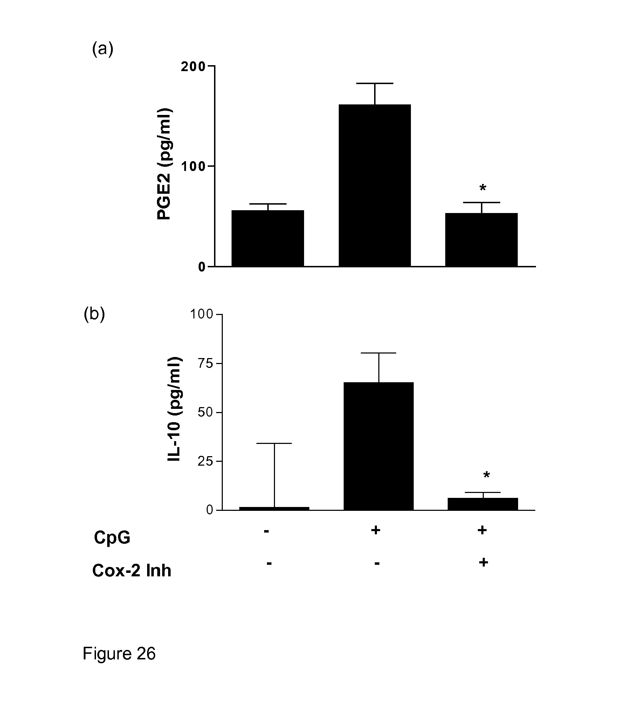

Bone marrow-derived DC were stimulated with CpG (5 µg) alone or in the presence of the Cox-2 inhibitor, NS-398. After 24 hours, supernatants were removed and concentrations of PGE₂ (Fig. 26(a)) were determined by ELISA. ^{*} p<0.05 CpG + p38 inhibitor versus CpG by ANOVA.

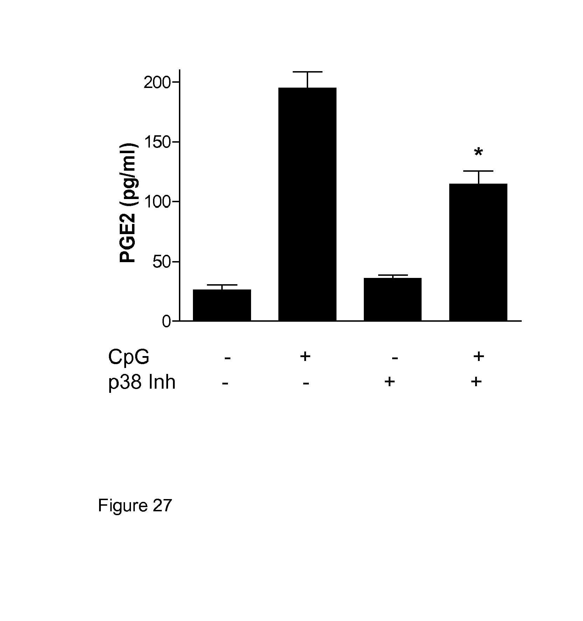

Bone marrow-derived DC were treated with heat shocked and irradiated B16 tumour cells for 4 hours, and then stimulated with CpG (5 µg), the p38 inhibitor SB 203580 (5 µM) or both for 24 hours. Supernatants were removed and PGE₂ concentrations determined by ELISA. ^{*} p<0.05 CpG + p38 inhibitor versus CpG by ANOVA (Fig. 27).

### Results

Fig. 25 shows that suppression of ERK activation reverses CpG-induced PGE2 production by dendritic cells.
Figure 26(a) show that suppression of Cox-2 production reverses CpG-induced PGE2 production by dendritic cells.
Figure 27 shows that suppression of p38 MAP kinase activation reverses CpG-induced PGE2 production by dendritic cells.

### Example 15 - Cox-2 inhibitor suppresses TLR agonist induced IL-10 production by dendritic cells

### Material and Methods

Bone marrow-derived DC were stimulated with CpG (5 µg) alone or in the presence of the Cox-2 inhibitor, NS-398. After 24 hours, supernatants were removed and concentrations of IL-10 (Fig. 26(b)) were determined by ELISA. ^{*} p<0.05 CpG + p38 inhibitor versus CpG by ANOVA.

### Results

Figure 26(b) show that suppression of Cox-2 production reverses CpG-induced IL-10 production by dendritic cells.

### Example 16 - Inhibition of p38 suppresses IL-10 and enhances IL-12 production by tumour vaccine and TLR agonist-stimulated dendritic cells

### Material and Methods

Bone marrow-derived DC were for 4 hours with B16 tumour cells that had been heat shocked and irradiated B16 tumour cells for 4 hours, and then stimulated with CpG (5 µg), the p38 inhibitor SB 203580 (5 µM) or both. Supernatants were removed after 24 hours and IL-10, IL-12p40, IL-12p70 and IL-23 concentrations were determined by ELISA. ^{*} p<0.05; ^{***} p<0.0001, CpG + p38 inhibitor versus CpG by ANOVA.

### Results

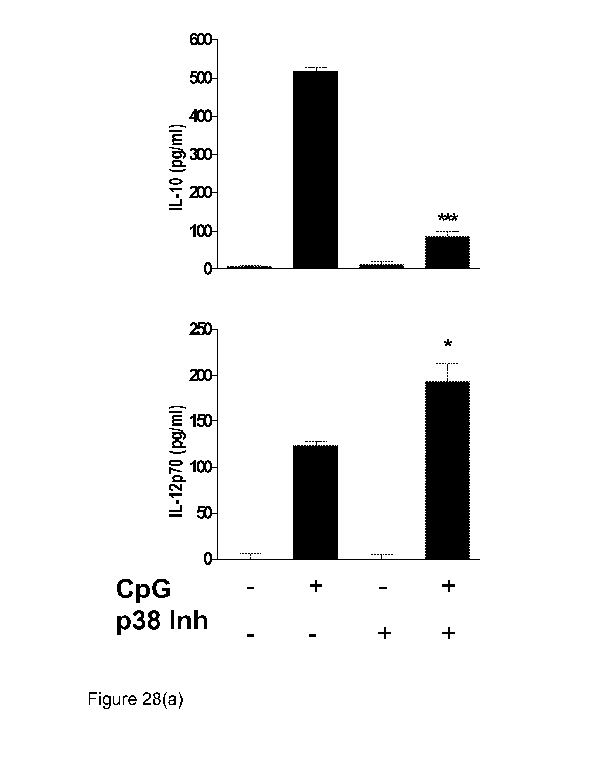

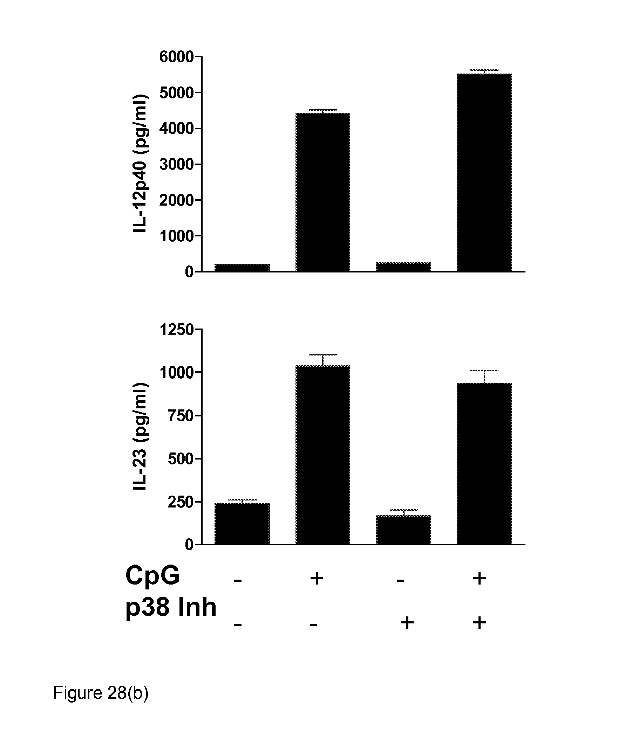

Figure 28 shows that suppression of p38 MAP kinase activation reverses CpG-induced IL-10, while enhancing IL-12 production by dendritic cells. IL-23 production was not affected by the p38 inhibitor.

### Example 17 - Inhibition of p38 enhances therapeutic efficacy of a dendritic cell vaccine and of dendritic cells pulsed with a tumour vaccine and CpG

### Material and Methods

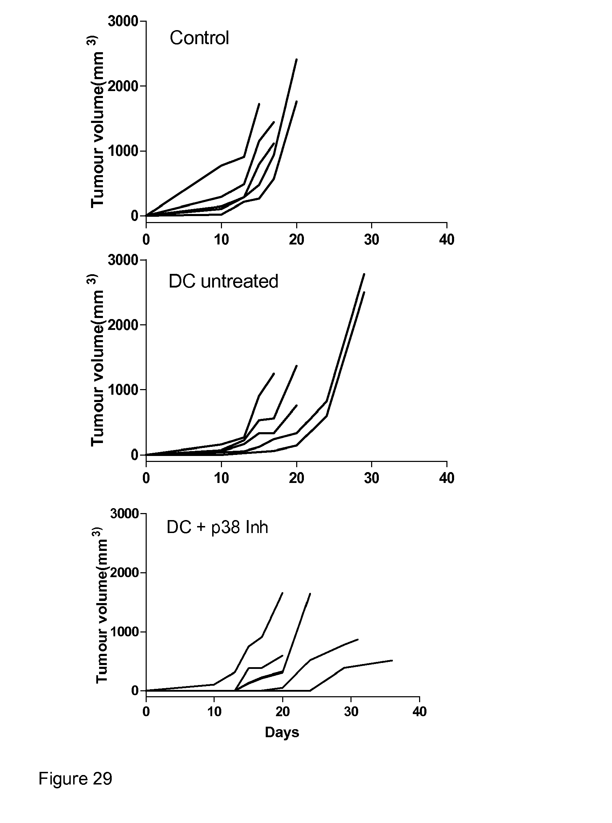

Bone marrow derived DC were treated for 4 hours with B16 tumor cells that had been heat shocked and irradiated B16 tumour cells for 4 hours, and then stimulated with medium only or the p38 inhibitor, SB 203580 (5 µM) for 24 hours. C57BL/6 mice were challenged with 2 x 10⁵ B16 tumour cells subcutaneously (s.c.) and treated on days 3, 10 and 17 days post challenge with 5 x 10⁵ DC by injection into the tumour site. Tumour growth was routinely monitored. Results represent tumour volumes for individual mice (Fig. 29).

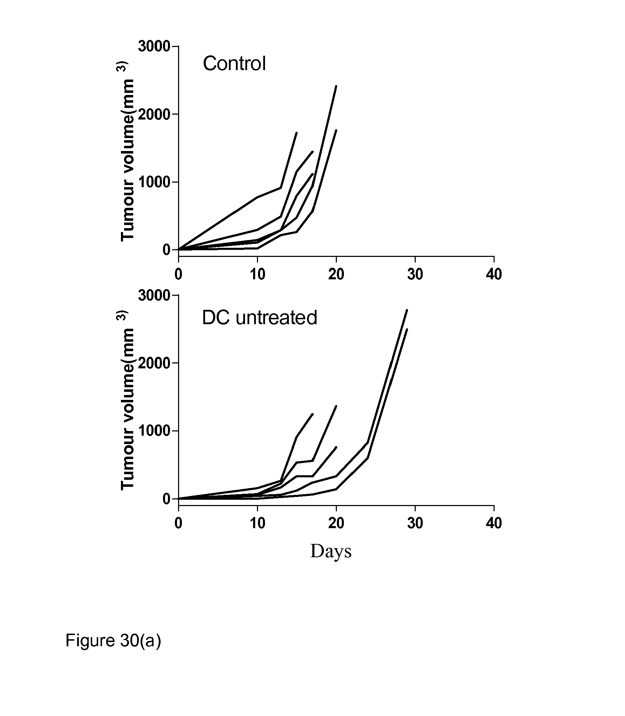

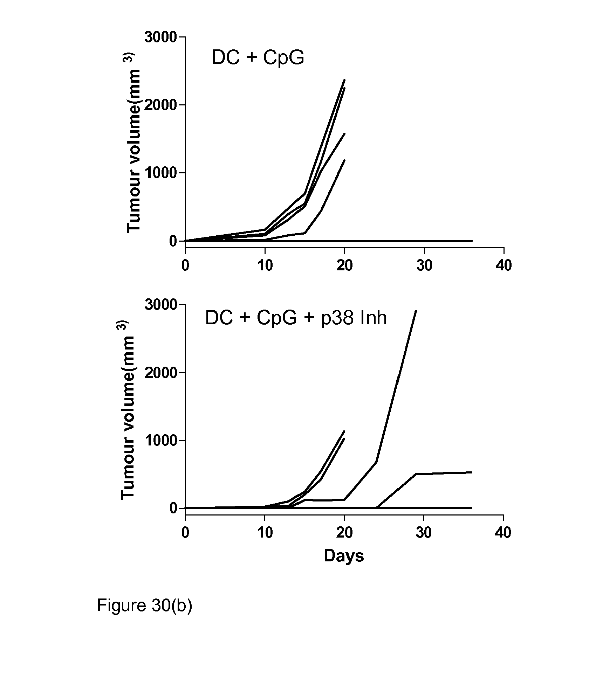

Bone marrow derived DC were treated for 4 hours with B16 tumor cells that had been heat shocked and irradiated B16 tumour cells for 4 hours, and then stimulated with either CpG (5 µg) alone or in the presence of the p38 inhibitor, SB203580 (5 µM) for 24 hours. C57BL/6 mice were challenged with 2 x 10⁵ B16 tumour cells s.c. and treated on days 3, 10 and 17 days post challenge with 5 x 10⁵ DC by injection into the tumour site. Tumour growth was routinely monitored. Results represent tumour volumes for individual mice (Fig. 30).

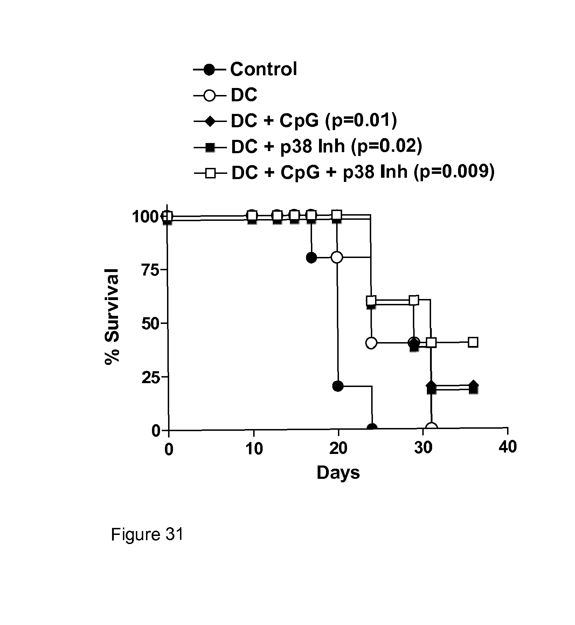

Bone marrow derived DC were treated with heat shocked/irradiated B16 tumour cells for 4 hours, and then stimulated with medium only, CpG (5 µg), CpG and the p38 inhibitor SB 203580 (5 µM) or the p38 inhibitor only for 24 hours. C57BL/6 mice were challenged with 2 x 10⁵ B16 tumour cells s.c. and treated on days 3, 10 and 17 days post challenge with 5 x 10⁵ DC by injection into the tumour site. Mice were routinely monitored for survival. Numbers in parentheses represent statistical difference versus control group (Fig. 31).

### Results

Figure 29 shows that a p38 inhibitor enhances the protective efficacy of a tumour vaccine comprising dendritic cells activated with killed whole tumour cells.
Figure 30 shows that a p38 inhibitor enhances the protective efficacy of tumour vaccine comprising dendritic cells activated with killed whole tumour cells in the presence of the TLR agonist CpG.
Figure 31 shows that a p38 inhibitor enhances survival of mice following therapeutic administration of a vaccine comprising dendritic cells activated with killed whole tumour cells alone or in the presence of the TLR agonist CpG.

### Example 18 - Therapy with dendritic cells pulsed with a whole tumour vaccine and CpG enhances T cell IFN-qamma production in the tumour.

### Material and Methods

Bone marrow derived DC were treated with heat shocked/irradiated B16 tumour cells for 4 hours, and then stimulated with medium only or CpG (5 µg) for 24 hours. C57BL/6 mice were challenged with 2 x 10⁵ B16 tumour cells s.c. and treated on days 3 and 10 days post challenge with 5 x 10⁵ DC by injection into the tumour site.

Tumours were extracted on day 15 and intracellular IL-17 and IFN-gamma in CD4⁺ and CD8⁺ T cells was determined by immunofluorescence analysis using a FACS.

### Results

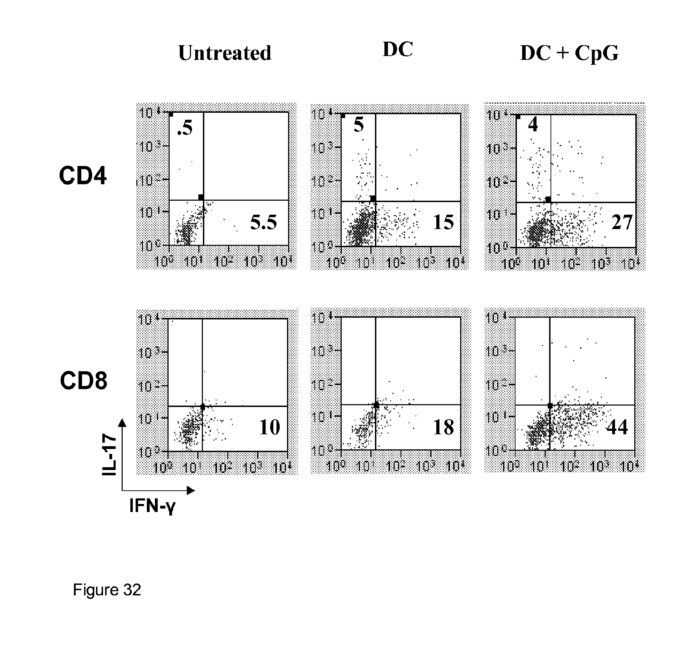

Figure 32 shows that therapeutic administration of dendritic cells pulsed with killed tumour cells and CpG to mice with s.c. tumours enhances the frequency of IFN-gamma producing by CD4⁺ and CD8⁺ T cells in the growing tumour.

### Example 19 - Therapy with dendritic cells treated with B16 vaccine, CpG and a p38 inhibitor enhances the frequency of CD4⁺ T cells but decreases the frequency of regulatory T cells within the tumour mass

### Material and Methods

Bone marrow derived DC were treated with heat shocked/irradiated B16 tumour cells for 4 hours, and then stimulated with medium only, CpG (5 µg), CpG and the p38 inhibitor SB 203580 (5 µM) or the p38 inhibitor only for 24 hours. C57BL/6 mice were challenged with 2 x 10⁵ B16 tumour cells s.c. and treated on days 3 and 10 days post challenge with 5 x 10⁵ DC by injection into the tumour site. Tumours were extracted on day 15 and the percentage of CD4⁺ cells (A) and CD4⁺CD25⁺Foxp3⁺ cells (B) in the tumour mass was determined by immunofluorescence with specific antibodies and analysis by FACS.

### Results

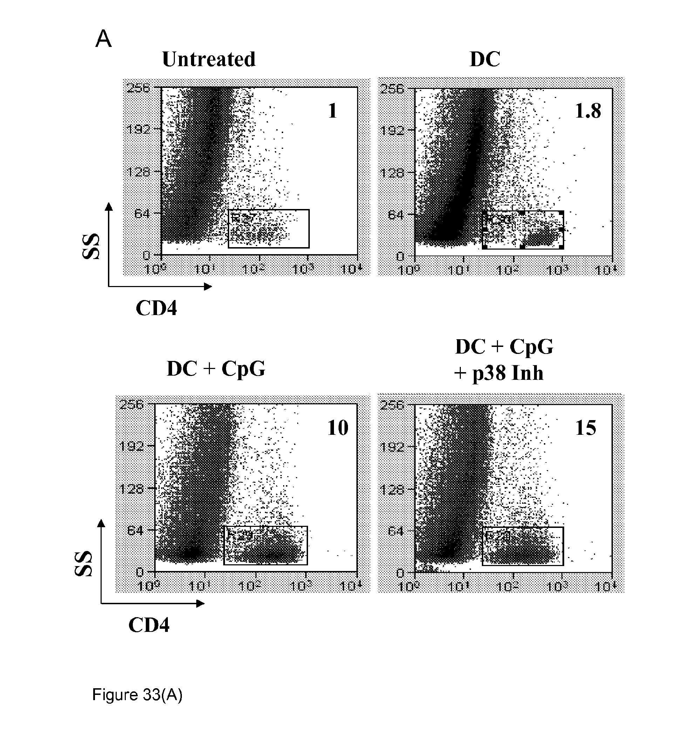

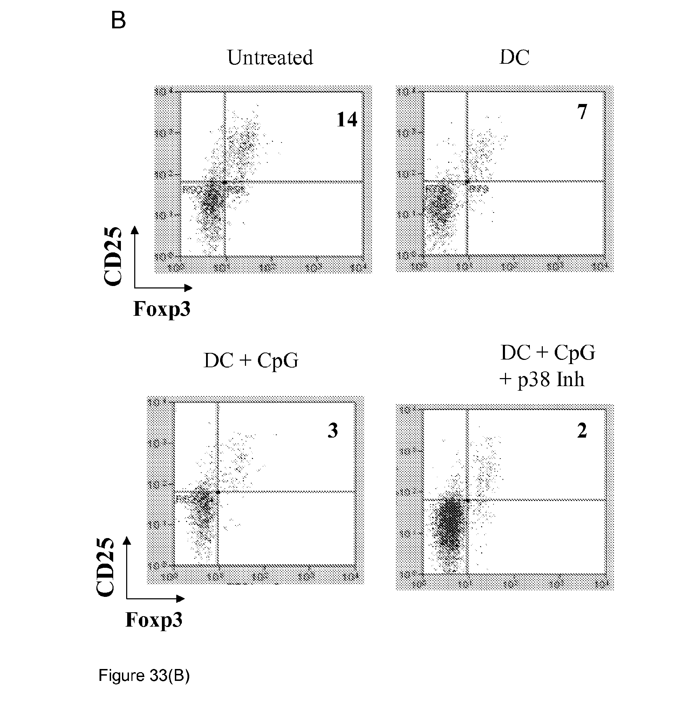

Figure 33 shows that therapeutic administration of dendritic cells pulsed with killed tumour cells and CpG with a p38 inhibitor to mice with s.c. tumours enhances the recruitment of CD4⁺ T cells into the growing tumour and suppresses the recruitment of Foxp3 expressing regulatory T cells.

### Example 20 - Pertumoural injection of CpG and a p38 inhibitor increases survival

### Material and Methods

C57BL/6 mice were challenged with 2 x 10⁵ B16 tumour cells s.c. and injected with CpG (25 µg), the p38 inhibitor SB203580 (50 µg) or both into the tumour site on days 3, 5 and 7 post challenge. Mice were routinely monitored for survival.

### Results

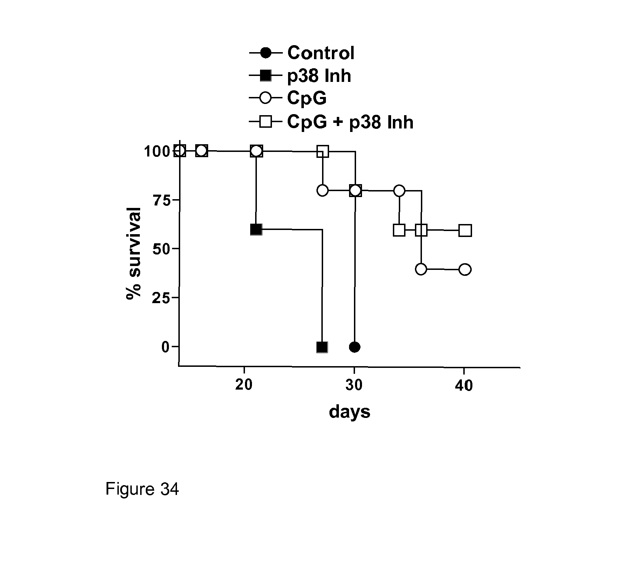

Figure 34 shows that therapeutic administration of CpG in the presence of a p38 inhibitor enhances survival of mice with s.c B16 tumours over that observed following therapy with CpG or p38 inhibitor alone.

### Example 21 - A pl3K inhibitor enhances therapeutic efficacy of dendritic cells pulsed with a tumour vaccine and CpG leading to enhanced survival of mice with growing tumours

### Material and Methods

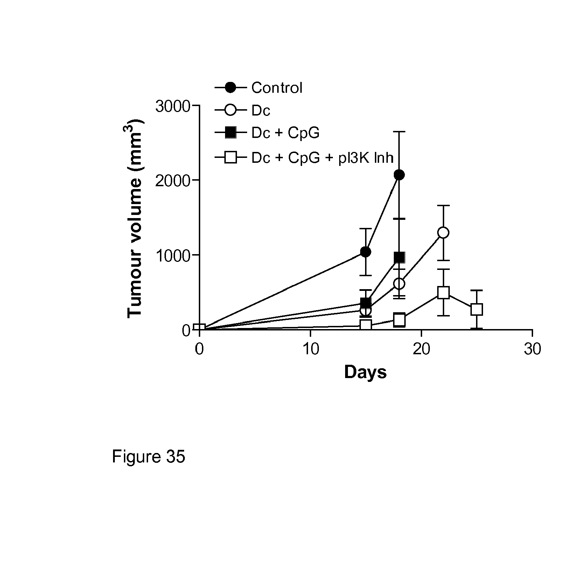

Bone marrow-derived DC were first pulsed in vitro with heat shocked and irradiated B16 tumour cells for 4 hours, and then stimulated with CpG (5 µg/ml) alone or with the pl3K inhibitor Wortmannin (2.5 µM) for 24 hours. C57BL/6 mice were challenged with 2 x 10⁵ B16 tumour cells s.c. and injected on days 3, 10 and 17 post challenge, into the tumour site with 5 x 10⁵ treated DC. Tumour growth was routinely monitored and the averages from each group plotted (Fig. 35).

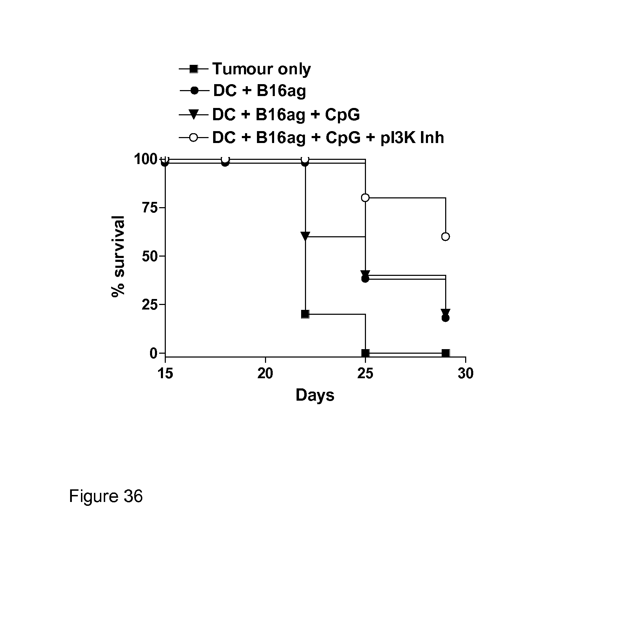

Bone marrow-derived DC were first pulsed in vitro with heat shocked and irradiated B16 tumour cells for 4 hours, and then stimulated with CpG (5 µg/ml) alone or with the pl3 kinase (pl3K) inhibitor Wortmannin (2.5 µM) for 24 h. C57BL/6 mice were challenged with 2 x 10⁵ B16 tumour cells s.c. and injected on days 3, 10 and 17 post challenge, into the tumour site with 5 x 10⁵ treated DC. Tumour growth was routinely monitored for survival (Fig. 36).

### Results

The results show that therapeutic administration of DC pulsed with killed tumour cells and CpG in the presence of a Pl3 kinase has greater therapeutic efficacy than DC pulsed with tumour cells and CpG or pl3K inhibitor alone (Figure 35), Furthermore, therapeutic administration of DC pulsed with killed tumour cells and CpG in the presence of a Pl3 kinase inhibitor enhanced survival of mice with s.c B16 tumours over that observed following therapy with DC pulsed with tumour cells and CpG or pl3K inhibitor alone (Figure 36).

### Example 22 - A p38 inhibitor with CpG as adjuvant enhances the protective efficacy of an acellular pertussis vaccine

### Material and Methods

Mice were immunized intraperitoneally (i.p.) twice (0 and 4 weeks) with 0.02 human dose of an acellular pertussis vaccine (JNIH-3; comprising FHA and detoxified pertussis toxin) alone or in the presence of CpG (5 µg) or CpG and the p38 inhibitor, SB203580 (50 µM). Mice were challenged with an aerosol of *Bordetella pertussis* 2 weeks later. The course of *B*. *pertussis* infection was followed by performing CFU counts on lungs from groups of 4 mice 0, 3, 7, 14 and 21 days after challenge. The lungs were aseptically removed and homogenised in 1 ml of sterile physiological saline with 1% casein on ice. Undiluted and serially diluted homogenate (100 µl) from individual lungs was spotted in triplicate onto Bordet-Gengou agar plates, and the number of CFU was calculated after 5 days incubation at 37°C. The limit of detection was approximately 0.6 log₁₀ CFU per lung.

### Results

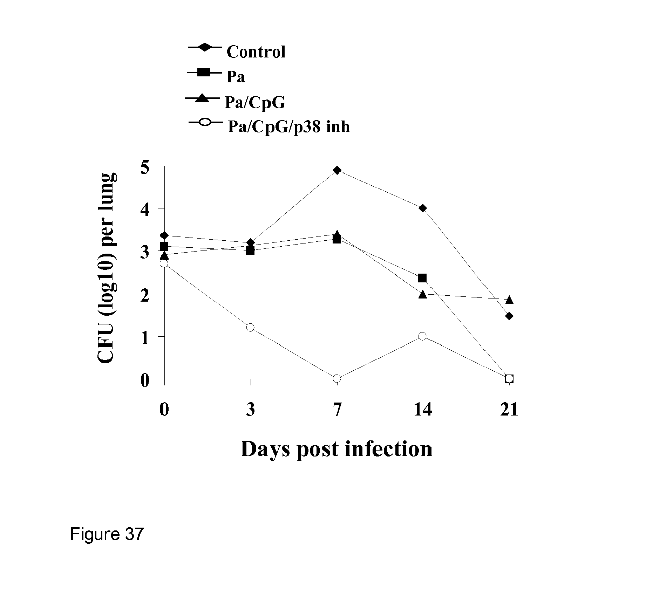

Figure 37 shows that a low dose of an acellular pertussis vaccine, even with the addition of CpG as adjuvant, is poorly protective in mice, but that co-administration of a p38 inhibitor significantly enhances the protective efficacy. There were significantly lower *B*. *pertussis* CFU in the lungs in mice immunized with the vaccine in the presence of CpG and the p38 inhibitor.

### Example 23 - A p38 inhibitor enhances IFN-gamma and reduces IL-10 in response to an acellular pertussis vaccine formulated with CpG as the adjuvant

### Material and Methods

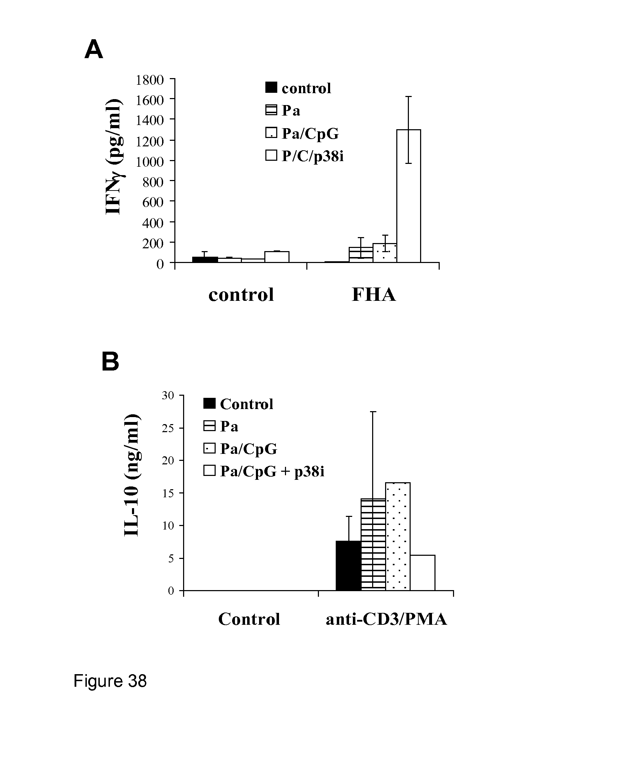

Mice were immunized parenterally twice (0 and 4 weeks) with 0.02 human dose of an acellular pertussis vaccine (JNIH-3 comprising FHA and detoxified pertussis toxin) alone or in the presence of CpG (5 µg) or CpG and the p38 inhibitor SB203580 (50µM). Mice were challenged with an aerosol of *Bordetella pertussis* 2 weeks later. Spleen mononuclear cells (2 x 10⁶/ml), removed before or after challenge, were cultured at 37°C and 5% CO₂ with purified filamentous hamagglutinin (FHA) from *B*. *pertussis* or with PMA (250 ng/ml; Sigma) and anti-mouse CD3 (1 µg/ml; Pharmingen, SanDiego, USA) or medium only as a negative control. Supernatants were removed after 72 hours and IL-10 and IFN-gamma concentrations determined by two-site ELISA (Fig. 38).

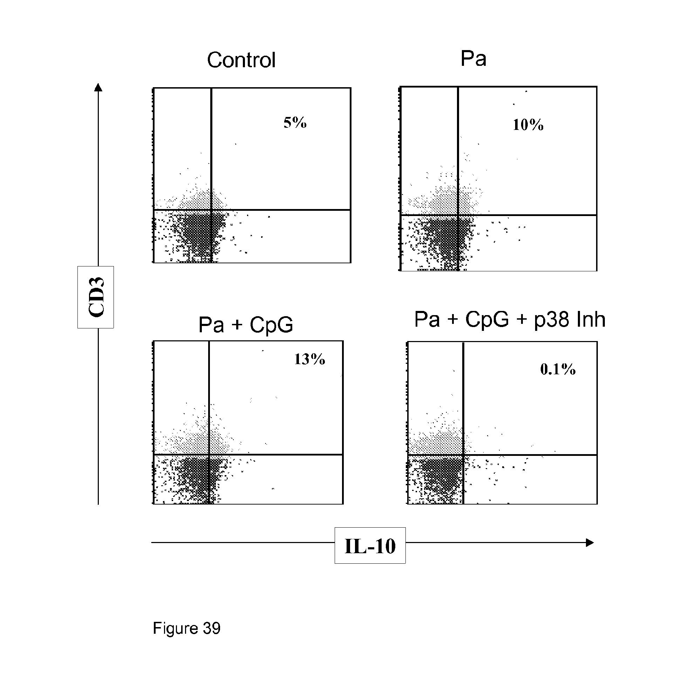

Mice were immunized i.p. twice (0 and 4 weeks) with an acellular pertussis vaccine (JNIH-3 comprising FHA and detoxified pertussis toxin) alone or in the presence of CpG (5 µg) or CpG and the p38 inhibitor, SB203580 (50µM). Mice were challenged with an aerosol of *Bordetella pertussis 2* weeks later. Spleen mononuclear cells (2 x 10⁶/ml), removed 14 days after challenge, were cultured at 37°C and 5% CO₂ with PMA (250 ng/ml; Sigma) and inomycin (1 µg/ml) in the presence of Brefaldin A. After 4 hours intracellular IL-10 was determined by immunofluorescence analysis using specific antibodies and analyzed on a FACS (Fig. 39).

### Results

The results show that a p38 inhibitor enhanced IFN-gamma and reduced IL-10 responses following immunization with an acellular pertussis vaccine with CpG as the adjuvant (Fig. 38 and Fig. 39).

### Example 24 - Addition of a p38 inhibitor to an acellular pertussis vaccine formulated with CpG as the adjuvant enhances local IL-1beta after B. pertussis challenge of mice

### Material and Methods

Mice were immunized i.p. twice (0 and 4 weeks) with an acellular pertussis vaccine (JNIH-3 comprising FHA and detoxified pertussis toxin) alone or in the presence of CpG (5 µg) or CpG and the p38 inhibitor SB203580 (50µM)). Mice were challenged with an aerosol of *Bordetella pertussis 2* weeks later. 4 hours after challenge lungs were removed and homogenized and the concentrations of IL-1beta determined by ELISA.

### Results

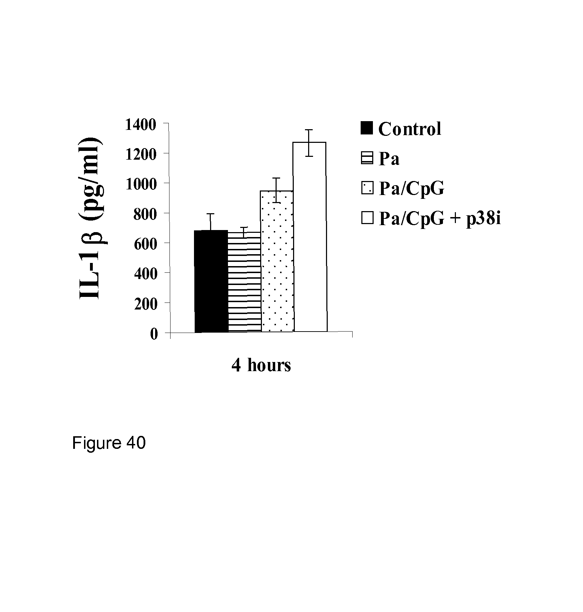

Figure 40 shows that inhibition of p38 during vaccination enhances the local inflammatory responses following *B. pertussis* challenge of immunized mice. Addition of a p38 inhibitor to an acellular pertussis vaccine formulated with CpG as the adjuvant enhanced local IL-1beta in the lungs after *B. pertussis* challenge.

### Example 25 - Therapeutic immunization with a tumor vaccine, TLR agonist and a p38 inhibitor reduces B16 tumor growth in mice.

C57BL/6 mice were challenged with 2 x 10⁵ B16 tumour cells s.c. and injected with vehicle only (un-treated) heat shocked and irradiated B16 tumor cells (1 x10⁶) alone or with CpG (10 µg) or with CpG and the p38 inhibitor SB203580 (50 µg) into the tumour site on days 3, 5 and 7 post challenge. Mice were routinely monitored for tumor growth.

### Results

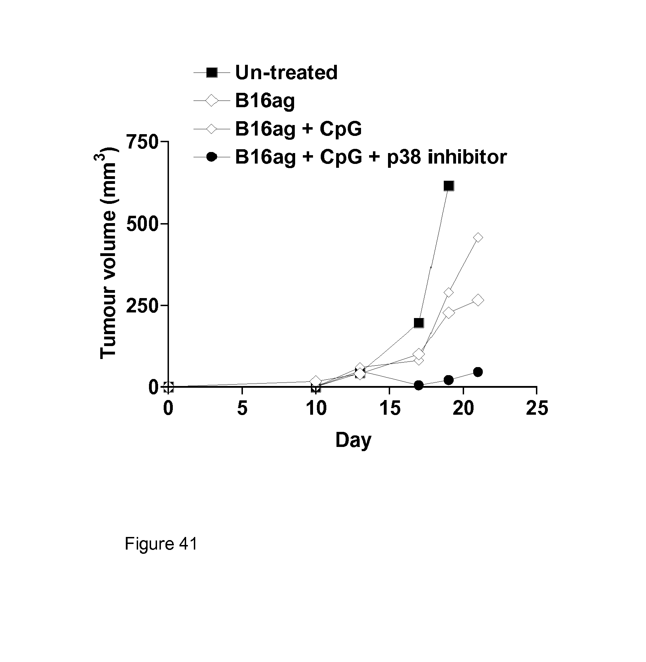

The results (Figure 41) show that therapeutic immunization with a killed tumor vaccine with CpG as adjuvant in the presence of a p38 inhibitor enhances survival of mice with s.c. B16 tumours over that observed following therapy with B16 vaccine alone or B16 vaccine and CpG.

### Example 26 - Tumour growth is not significantly exacerbated in IL-10-defective mice

C57BL/6 or IL-10-defetive mice were challenged with 2 x 10⁵ B16 tumour cells s.c. and 17 days later tumour sizes were evaluated. Results are mean tumor volumes for 5 mice per group.

### Results

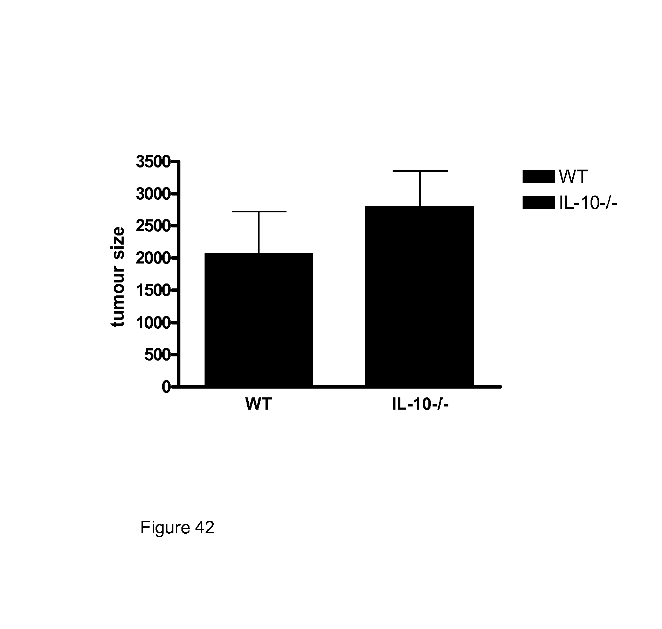

The results (Figure 42) show that tumour growth is not significantly exacerbated in IL-10-defective mice. Therefore IL-10 is shown not to be the sole mediator in mediating an anti-inflammatory response.

All documents referred to in this specification are herein incorporated by reference. Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. A composition for the treatment of a condition where an enhancement of a Th1-mediated immune response is desired, said composition comprising:
(i) at least one Toll-like receptor (TLR) agonist selected from the group comprising; Flagellin,Pam3CSK4, Zymosan, PolylC, dsRNA, LPS (lipopolysaccharide), monophosphoryl lipid A (MPL), CpG-ODN (CPG-oligodeoxynucleotides), Imiquimod, R838, R83, *Bordetella pertussis,* and *Mycobacterium tuberculosis;* and
(ii) at least one immune modulator which inhibits the suppression of an immune response, wherein said at least one immune modulator is selected from the group consisting of: a Phosphoinositide kinase-3 inhibitor, a cyclooxygenase 2 inhibitor, a MAP kinase protein a p38 kinase inhibitor, an ERK inhibitor, a MEK 1 inhibitor, and a MEK 2 inhibitor, and wherein this suppression results from the selective inhibition of function of regulatory T cells or from a modulation of cytokine expression such that at least one anti-inflammatory cytokine is suppressed and at least one pro-inflammatory cytokine is upregulated.

2. The composition as claimed in claim 1 or claim 2 wherein the immune modulator inhibits the production of IL-10 and/or TGF-beta and/or upregulates the production of IL-12.

3. The composition as claimed in claim 1 or claim 2 further comprising at least one tumour specific antigen.

4. The composition as claimed in any one of claims 1 to 3 further comprising a modulatory compound which inhibits a tumour cell product which functions to enhance tumour cell survival.

5. The composition as claimed in claim 4 wherein the modulatory compound is a tumour growth inhibitory product or modulatory compound which promotes the onset of apoptosis.

6. The composition as claimed in any one of claims 1 to 5 further comprising at least one tumour specific antigen against which an immune response can be mounted, said immune response being specific for the cancerous or malignant condition from which the tumour specific antigen is derived.

7. The composition as claimed in claim 6 wherein the tumour specific antigen is derived from a complex of a heat shock protein and antigenic peptide derived from a cancerous cell or an individual with a cancerous condition.

8. The composition as claimed in claim 6 or claim 7 further comprising a modulatory compound which inhibits a tumour cell product which functions to enhance tumour cell survival.

9. The composition as claimed in any one of claims 1 to 8 further comprising a dendritic cell.

10. Use of at least one Toll-like receptor agonist and an immune modulator which inhibits the suppression of an immune response through the selective inhibition of function of regulatory T cells or from the modulation of cytokine expression such that at least one anti-inflammatory cytokine is suppressed and at least one pro-inflammatory cytokine is upregulated in the preparation of a medicament for the treatment of condition where an enhancement of a Th1-mediated or other effector immune response is desired.

11. Use of a composition comprising at least one tumour specific antigen, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated, in the preparation of a medicament for the treatment of a cancerous or malignant condition.

12. Use of a dendritic cell, at least one tumour specific antigen, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated, in the preparation of a vaccine composition for the treatment of a cancerous condition.

13. Use of a dendritic cell, at least one Toll-like receptor agonist and an immune modulator compound which inhibits the suppression of an immune response through the selective inhibition of function of regulatory T cells or from the modulation of cytokine expression such that at least one anti-inflammatory cytokine is suppressed and at least one pro-inflammatory cytokine is upregulated in the preparation of a vaccine composition for the treatment of a cancerous condition.

14. Use as claimed in any one of claims 10 to 13 wherein the at least one Toll-like receptor agonist is Flagellin.

15. Use as claimed in any one of claims 10 to 14 wherein the immune modulator compound is a Phosphoinositide kinase-3 inhibitor.


SEQUENCE LISTING

<110>  The Provost, Fellows and Scholars of the College of the
       Holy and Undivided Trinity of Queen Elizabeth, near Dublin
       Mills, Kingston
       Toomey, Deirdre
       Jarnicki, Andrew

<120>  Compositions and methods relating to treatment of cancer and
       infectious diseases

<130>  P43803.WO.01/AMC/GST

<140>  PCT/IB2006/003951
<141>  2006-12-01

<150>  IE2005/0799
<151>  2005-12-01

<160>  16

<170>  PatentIn version 3.3

<210>  1
<211>  26
<212>  DNA
<213>  Artificial

<220>
<223>  primer specific for murine TGF-beta (sense)

<400>  1
agacggaata cagggctttc gattca                                          26


<210>  2
<211>  24
<212>  DNA
<213>  Artificial

<220>
<223>  primer specific for murine TGF-beta (anti-sense)

<400>  2
cttgggcttg cgacccacgt agta                                            24


<210>  3
<211>  24
<212>  DNA
<213>  Artificial

<220>
<223>  primer specific for IL-10 (sense)

<400>  3
ctggacaaca tactgctaac cgac                                            24


<210>  4
<211>  24
<212>  DNA
<213>  Artificial

<220>
<223>  primer specific for IL-10 (anti-sense)

<400>  4
ttcattcatg gccttgtaga cacc                                            24

<210>  5
<211>  21
<212>  DNA
<213>  Artificial

<220>
<223>  primer specific for Foxp3 (sense)

<400>  5
cagctgccta cagtgcccct a                                                21


<210>  6
<211>  20
<212>  DNA
<213>  Artificial

<220>
<223>  primer specific for Foxp3 (anti-sense)

<400>  6
catttgccag cagtgggtag                                                  20


<210>  7
<211>  24
<212>  DNA
<213>  Artificial

<220>
<223>  primer specific for Cox-2 (sense)

<400>  7
gtatcagaac cgcattgcct ctga                                             24


<210>  8
<211>  27
<212>  DNA
<213>  Artificial

<220>
<223>  primer specific for Cox-2 (anti-sense)

<400>  8
cggcttccag tattgaggag aacagat                                          27


<210>  9
<211>  22
<212>  DNA
<213>  Artificial

<220>
<223>  primer specific for IP-10 (sense)

<400>  9
cgcacctcca catagcttac ag                                               22


<210>  10
<211>  22
<212>  DNA
<213>  Artificial

<220>
<223>  primer specific for IP-10 (anti-sense)

<400>  10
cctatcctgc ccacgtgttg ag                                                22

<210>  11
<211>  19
<212>  DNA
<213>  Artificial

<220>
<223>  primer specific for IL-23p19 (sense)

<400>  11
tctcggaatc tctgcatgc                                                    19

<210>  12
<211>  20
<212>  DNA
<213>  Artificial

<220>
<223>  primer specific for IL-23p19 (anti-sense)

<400>  12
ctggaggagt tggctgagtc                                                   20

<210>  13
<211>  32
<212>  DNA
<213>  Artificial

<220>
<223>  primer specific for IL-15 (sense)

<400>  13
catatggaat ccaactggat agatgtaaga ta                                     32

<210>  14
<211>  30
<212>  DNA
<213>  Artificial

<220>
<223>  primer specific for IL-15 (anti-sense)

<400>  14
catatgctcg agggacgtgt tgatgaacat                                        30

<210>  15
<211>  23
<212>  DNA
<213>  Artificial

<220>
<223>  primer specific for beta-actin (sense)

<400>  15
ggactcctat gtgggtgacg agg                                               23

<210>  16
<211>  21
<212>  DNA
<213>  Artificial

<220>
<223>  primer specific for beta-actin (anti-sense)

<400>  16
tgccaatagt gatgacttgg c                                                21